# EUROPEAN PATENT APPLICATION

(11) **EP 3 079 037 A1**
(43) Date of publication of application: **12.10.2016**
(21) Application number: 14867451.8
(22) Date of filing: 03.10.2014
(51) Int. Cl.: G06F 1/26, H02J 7/35

(54) **ELECTRONIC DEVICE, INFORMATION PROCESSING METHOD, AND INFORMATION PROCESSING SYSTEM**

(30) Priority: 05.12.2013 JP 2013252033
(71) Applicant: Sony Corporation, Tokyo 108-0075 (JP)
(72) Inventor: YAJIMA, Masakazu, Tokyo 108-0075 (JP)
(74) Representative: Witte, Weller & Partner Patentanwälte mbB
(86) International application number: PCT/JP2014/005053
(87) International publication number: WO 2015/083311

(57) **Abstract**

For example, an electronic device includes: a power generating unit configured to generate electric power according to a surrounding environment; a state transition unit configured to cause a state to transition according to the electric power supplied from the power generating unit; and an output unit configured to output predetermined information according to the transition of the state of the state transition unit.

## Description

### Technical Field

The present disclosure relates to an electronic device, an information processing method, and an information processing system.

### Background Art

An electronic device that is worn on a human body and obtains various kinds of information is known. For example, Patent Literature 1 discloses a momentum meter including an acceleration sensor.

### Citation List

### Patent Literature

Patent Literature 1: JP 2009-22440A

### Summary of Invention

### Technical Problem

In the electronic device disclosed in Patent Literature 1 or the like, since a plurality of sensors and an analog to digital (AD) converter connected thereto are used to obtain ample information, there is a problem in that electric power consumed in the electronic device is increased.

It is an object of the present disclosure to provide an electronic device, an information processing method, and an information processing system, which are capable of solving the above problem.

### Solution to Problem

To solve the above described problem, for example, the present disclosure provides an electronic device including: a power generating unit configured to generate electric power according to a surrounding environment; a state transition unit configured to cause a state to transition according to the electric power supplied from the power generating unit; and an output unit configured to output predetermined information according to the transition of the state of the state transition unit.

For example, the present disclosure provides an electronic device including: a power storage unit configured to accumulate a first energy; a state transition unit configured to cause a state to transition according to the first energy; a converting unit configured to convert the first energy into a second energy; and an output unit configured to output predetermined information using the second energy, the second energy being supplied according to the transition of the state of the state transition unit.

For example, the present disclosure provides an information processing method in an electronic device, the information processing method including: generating, by a power generating unit, electric power according to a surrounding environment; causing, by a state transition unit, a state to transition according to the electric power supplied from the power generating unit; and outputting, by an output unit, predetermined information according to the transition of the state of the state transition unit.

For example, the present disclosure provides an information processing system including: a first electronic device; and a second electronic device. The first electronic device includes a power generating unit configured to generate electric power according to a surrounding environment, a state transition unit configured to cause a state to transition according to the electric power supplied from the power generating unit, and an output unit configured to output predetermined information to the second electronic device according to the transition of the state of the state transition unit. The second electronic device includes an acquiring unit configured to acquire the predetermined information output from the first electronic device.

### Advantageous Effects of Invention

According to at least one embodiment, it is possible to provide an electronic device in which power consumption is small. The effect described herein is not necessarily limited, and any effect described in the present disclosure may be included. Further, the content of the present disclosure should not be interpreted as limited by the exemplified effect.

Brief Description of Drawings

[FIG. 1] FIG. 1 is a block diagram for describing an example of a configuration of an electronic device.
[FIG. 2] FIG. 2 is a block diagram for describing an example of a configuration of a module in an electronic device according to a first embodiment.
[FIG. 3] FIG. 3 is a diagram for describing an example of a system using an electronic device.
[FIG. 4] FIG. 4 is a flowchart illustrating an example of the flow of a process of the electronic device according to the first embodiment.
[FIG. 5] FIG. 5 is a flowchart illustrating a modified example of the flow of the process of the electronic device according to the first embodiment.
[FIG. 6] FIG. 6 is a flowchart illustrating a modified example of the flow of the process of the electronic device according to the first embodiment.
[FIG. 7] FIG. 7 is a block diagram for describing an example of a configuration of a module in an electronic device according to a second embodiment.
[FIG. 8] FIG. 8 is a flowchart illustrating an example of the flow of a process of the electronic device according to the second embodiment.
[FIG. 9] FIG. 9 is a block diagram for describing an example of a configuration of a module in an electronic device according to a third embodiment.
[FIG. 10] FIG. 10 is a flowchart illustrating an example of the flow of a process of the electronic device according to the third embodiment.
[FIG. 11] FIG. 11 is a diagram for describing an example of an external appearance of an electronic device.
[FIG. 12] FIG. 12 is a diagram for describing an example of an internal configuration of a housing of an electronic device.
[FIG. 13] FIG. 13 is a diagram for describing an example of a physical configuration of an electronic device.
[FIG. 14] FIG. 14 is a diagram for describing an example of an extension module.
[FIG. 15] FIG. 15 is a diagram for describing a main communication module as an example of an extension module.
[FIG. 16] FIG. 16 is a diagram for describing an example of a configuration of a communication system.
[FIG. 17] FIG. 17 is a diagram for describing another example of a configuration of a communication system.
[FIG. 18] FIG. 18 is a diagram for describing another example of a configuration of a communication system.
[FIG. 19] FIG. 19 is a diagram for describing another example of a configuration of a communication system.
[FIG. 20] FIG. 20 is a diagram for describing another example of a configuration of a communication system.
[FIG. 21] FIG. 21 is a diagram for describing an application example of an electronic device.
[FIG. 22] FIGS. 22A to 22D are diagrams for describing application examples of an electronic device.
[FIG. 23] FIGS. 23A to 23D are diagrams for describing application examples of an electronic device.
[FIG. 24] FIG. 24 is a diagram for describing a modified example.
[FIG. 25] FIG. 25 is a diagram for describing a modified example.

### Description of Embodiments

Hereinafter, a plurality of embodiments of the present disclosure will be described with reference to the appended drawings. The description will proceed in the following order.
<1-1. First embodiment>
<1-2. Modified example of first embodiment>
<2. Second embodiment>
<3. Third embodiment>
<4. Content common to embodiments>
<5. Application examples>
<6. Modified examples>

Embodiments which will be described below are exemplary examples of the present disclosure, and the content of the present disclosure is not limited to the following embodiments.

### <1-1. First embodiment>

### <Example of configuration of electronic device>

First of all, an example of a configuration of an electronic device according to the first embodiment will be described. For example, the electronic device is a device that deals with a power generation state as sensing information. For example, an electronic device according to the embodiment is a small device that can be worn on an organism such as a person or an animal. Of course, an electronic device according to the embodiment may be a stationary device or one installed in various kinds of devices.

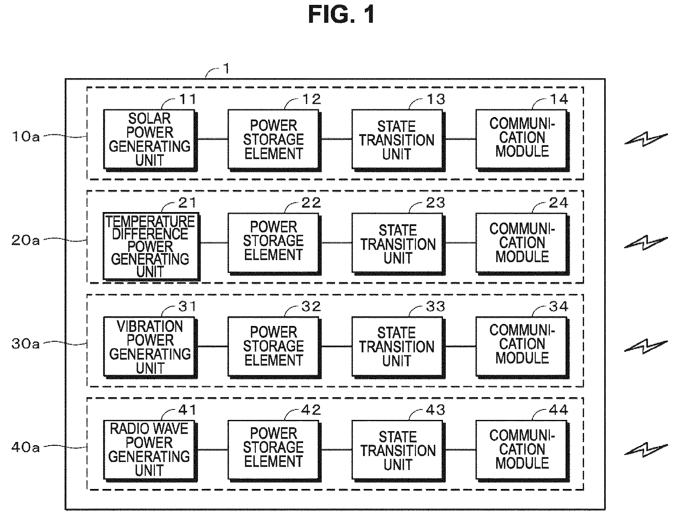

FIG. 1 illustrates an example of a schematic configuration of the electronic device according to the first embodiment. An electronic device 1 includes, for example, one or more of modules. As illustrated in FIG. 1, the electronic device 1 includes, for example, four modules (a module 10a, a module 20a, a module 30a, and a module 40a). The modules include at least a power generating unit that generates electric power according to a surrounding environment, a state transition unit that causes a state to transition according to the electric power supplied from the power generating unit, and an output unit that outputs predetermined information according to the transition of the state of the state transition unit.

The power generating unit generates electric power based on energy existing in a surrounding environment. The power generating unit, for example, generates electric power based on vibrations or motion of the user of the electronic device. For example, an electrostatic type, an electromagnetic type, an inverse magnetostrictive type, or a piezoelectric type may be used as a power generation method, and there is no preference for a power generation method. The power generating unit may generate electric power based on light (for example, an indoor electric bulb or sunlight. The power generating unit may be a thermoelectric conversion element (for example, an element that generates electric power using a Seebeck effect or a Thomson effect, a thermoelectric power generation element, or an element that performs thermomagnetic power generation) that generates electric power using a temperature difference. The power generating unit may be an enzyme battery cell (also referred to as a "bio-battery cell") that generates electric power using sugar. The power generating unit may generate electric power using capacity coupling or electromagnetic coupling by any one or a combination of inductance, capacitance, and reactance (LCR) components, a capacitor, an antenna, a rectenna, and the like, for example, using a radio wave. The power generating unit may perform near electromagnetic field power generation, that is, may generate electric power based on energy obtained by getting the electronic device close to a predetermined device. A known scheme such as a magnetic field resonance scheme, an electromagnetic induction scheme, electrolytic coupling, or an electric field resonance scheme may be applied as a near electric filed power generation scheme. In addition to the exemplified elements, any other known power generation element may be applied as the power generating unit.

The modules of the electronic device 1 may include one or more power storage elements. The power storage element is used, for example, according to the purpose of storing the electric power generated by the power generating unit. As the power storage element, in addition to various kinds of secondary battery cells such as a lithium-ion secondary battery cell, an electric double layer capacitor, a lithium-ion capacitor, a polyacene-based organic semiconductor (a polyacenic semiconductor (PAS)) capacitor, a Nanogate capacitor ("Nanogate" is a registered trademark of Nanogate Aktiengesellschaft), a ceramic capacitor, a film capacitor, an aluminum electrolytic capacitor, a tantalum capacitor, or the like may be used. A combination of power storage elements may be used according to the purpose.

The state transition unit causes its state to transition according to the electric power supplied from the power generating unit. The electric power supplied from the power generating unit may be supplied to the state transition unit directly or through the power storage element. The electric power generated by the power generating unit may be appropriately increased or decreased and then supplied to the state transition unit.

The state transition unit is configured with, for example, an integrated circuit (IC) including one or more elements. Examples of the state transition unit include a switching element such as a transistor, a diode, a reset IC, a regulator IC, a logic IC, and various kinds of operation circuits. A circuit configuration in an IC may be appropriately changed as long as the function of the state transition unit can be implemented. In the following description, a reset IC is assumed to be used as the state transition unit.

For example, the state transition unit transitions between two states of ON and OFF according to the electric power supplied from the power generating unit. For example, the state transition unit transitions from an OFF state to an ON state when the power generation amount output from the power generating unit is a predetermined amount or more. For example, the power generation amount is specified by any one of a voltage, an electric current, electric power and electric energy or a combination thereof. Further, when the electric power of the power generating unit is supplied to the state transition unit through the power storage element, the state transition unit transitions from the OFF state to the ON state when the power generation amount charged in the power storage element is the predetermined amount or more.

The state transition unit may transition among three or more states. The state transition unit preferably stores a state after transition by holding the state, but the state transition unit may neither hold nor store the state due to a reset or the like.

The output unit outputs the predetermined information depending on the state the state transition unit transitions to. For example, the output unit includes a communication module and an antenna for performing communication with an external device different from the electronic device 1, and performs communication based on a predetermined communication standard to output the predetermined information to the external device. The state transition unit and the communication module may be connected to a control unit, and the communication module may operate according to control by the control unit. The communication module may be configured to include the control unit.

The communication performed by the communication module may be wireless or wired communication. The wireless communication may be communication using an electromagnetic wave (including infrared rays) or communication using an electric field. As a specific scheme, a communication using a band of several hundreds of megahertz (MHz) to several gigahertz (GHz) such as "Wi-Fi (a registered trademark)," "Zigbee (a registered trademark)," "Bluetooth (a registered trademark)," "Bluetooth low energy," "ANT (a registered trademark)," "ANT+ (a registered trademark)," or "Enocean (a registered trademark)" may be used. Near field communication (NFC) may be used.

For example, when the state transition unit transitions to the ON state, the output unit operates, and communication is performed. The predetermined information output by the output unit is, for example, an identifier (ID) allocated to each module. In addition to the identifier, the predetermined information may be information of one bit (0 or 1 as a logical meaning) corresponding to the state of the state transition unit. For example, when the modules include different power generating units, the allocation of the identifier to each module has an equivalent meaning to the allocation of an identifier to each power generating unit.

The identifier allocated to the module may be an identifier that is allocated in advance or may be an identifier that is allocated each time. For example, when the electronic device 1 establishes a communication connection with another device, the identifier may be allocated to each module so that the allocated identifier is used.

In this example, the communication modules of the respective modules are configured to perform communication independently. Thus, it is unnecessary to control, for example, a timing at which each communication module performs communication.

### <Configuration of module in electronic device>

As illustrated in FIG. 1, the module 10a includes a solar power generating unit 11 that generates electric power using irradiated sunlight as an example of a power generating unit. The module 10a further includes a power storage element 12 connected to the solar power generating unit 11, a state transition unit 13 connected to the power storage element 12, and a communication module 14 connected to the state transition unit 13.

The module 20a includes a temperature difference power generating unit 21 that generates electric power using a temperature difference as an example of a power generating unit. The module 20a further includes a power storage element 22 connected to the temperature difference power generating unit 21, a state transition unit 23 connected to the power storage element 22, and a communication module 24 connected to the state transition unit 23.

The module 30a includes a vibration power generating unit 31 that generates electric power according to vibrations as an example of a power generating unit. The module 30a further includes a power storage element 32 connected to the vibration power generating unit 31, a state transition unit 33 connected to the power storage element 32, and a communication module 34 connected to the state transition unit 33.

The module 40a includes a radio wave power generating unit 41 that generates electric power using a radio wave as an example of a power generating unit. The module 40a further includes a power storage element 42 connected to the radio wave power generating unit 41, a state transition unit 43 connected to the power storage element 42, and a communication module 44 connected to the state transition unit 43. As described above, in the electronic device 1, the state transition unit and the output unit are installed for each power generating unit as an example.

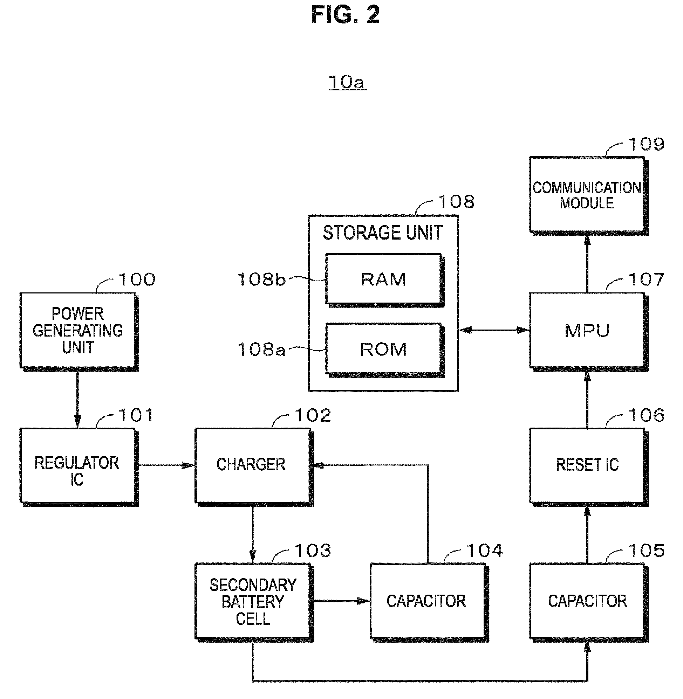

An example of a specific configuration of the module will be described in connection with an example of the module 10a. FIG. 2 is an example of a specific configuration of the module 10a. The module 10a has a configuration including, for example, a power generating unit 100, a regulator IC 101, a charger 102, a secondary battery cell 103, a capacitor 104, a capacitor 105, a reset IC 106, an MPU 107, a storage unit 108, and a communication module 109. The storage unit 108 has a configuration including, for example, a read only memory (ROM) 108a and a random access memory (RAM) 108b.

As described above, in the case of the module 10a, the power generating unit 100 is the solar power generating unit 11 including a solar cell. The solar power generating unit 11 may be configured such that, for example, a solar power generating unit using a dye-sensitized solar cell and a solar power generating unit using an amorphous silicon (Si) solar cell which employs the same power generation principle for sunlight power generation are separately installed.

The electric power generated by the power generating unit 100 is supplied to the regulator IC 101. The regulator IC 101 increases or decreases an input voltage so that an output voltage is constant. The regulator IC 101 increases or decreases the input voltage according to the configuration of the power generating unit 100. The output voltage of the regulator IC 101 is supplied to the charger 102.

The charger 102 is a circuit that charges the secondary battery cell 103 using the electric power supplied from the regulator IC 101. The charger 102 may be configured to control the charging of the secondary battery cell 103. The charger 102 may be configured to monitor the presence or absence of an abnormality of the secondary battery cell 103. The secondary battery cell 103 is charged according to the control of the charging by the charger 102.

The secondary battery cell 103 is a rechargeable battery cell. For example, a lithium-ion secondary battery cell may be used as the secondary battery cell 103. Of course, any other secondary battery cell may be used. Since the capacity of the secondary battery cell 103 is, for example, several milliwatt hours (mWh), the size of the secondary battery cell 103 need not be increased. An output voltage of the secondary battery cell 103 is supplied to the capacitor 104 and the capacitor 105.

The capacitor 104 is charged by the output voltage of the secondary battery cell 103. The electric power charged in the capacitor 104 is used as electric power of the charger 102. A power source for operating the charger 102 may be separately installed.

The capacitor 105 is charged by the output voltage of the secondary battery cell 103. The capacitor 105 is installed to extract, for example, a weak electric current. Further, when the reset IC 106 is used as the state transition unit using the capacitor 105, the reset IC 106 or the like can perform an operation according to the voltage of the capacitor 105, and thus the configuration of the circuit can be simplified. Instead of installing the capacitor 105, the secondary battery cell 103 may be connected to the reset IC 106. The secondary battery cell 103, the capacitor 104, and the capacitor 105 correspond to an example of the power storage element 12.

The reset IC 106 is an example of the state transition unit 13. For example, the reset IC 106 includes elements such as a comparator for comparing the voltage of the capacitor 105 with a reference voltage and a transistor that is turned on or off according to the comparison result. For example, the reference voltage is set to be equal to or higher than an operation voltage (3.3 V, 5 V, or the like) of the MPU 107.

The reset IC 106 transitions from the OFF state to the ON state when the voltage of the capacitor is the reference voltage or higher. As the reset IC 106 transitions to the ON state, the capacitor 105 is connected to the MPU 107. Then, the electric power charged in the capacitor 105 is supplied as the operation voltage of the MPU 107.

The MPU 107 serving as an example of the control unit is connected to the reset IC 106 and the communication module 109. The MPU 107 operates using electric power supplied as the reset IC 106 transitions to the ON state as its power source. The MPU 107 controls the communication module 109 and the like.

The storage unit 108 connected to the MPU 107 includes, for example, the ROM 108a and the RAM 108b. The ROM 108a stores, for example, a program performed by the MPU 107. The ROM 108a may store an identifier that is allocated to the module 10a in advance. The RAM 108b is used as a work memory when the MPU 107 performs a process. For example, when the identifier of the module 10a is allocated to establish a communication connection, the allocated identifier may be stored in the RAM 108b.

The communication module 109 performs a process based on a predetermined communication scheme according to control by the MPU 107. Although not illustrated, the communication module 109 includes a small antenna, such as a film antenna or a bar antenna, or a capacitor sufficiently satisfying an antenna function. As described above, a known scheme can be applied as a communication scheme performed by the communication module 109, and the communication scheme performed by the communication module 109 is not limited to a specific communication scheme. The storage unit 108 may be connected to the communication module 109. A plurality of storage units (for example, two storage units) may be connected to the MPU 107 and the communication module 109, respectively.

The example of the configuration of the module 10a has been described above, but configurations of the other modules may be implemented by appropriately changing the configuration of the power generating unit. For example, the state transition unit of the module 10a may have a different configuration from the state transition unit of the module 20a. In the different configuration mentioned above, for example, when the output of the power generating unit is alternating current (AC) power, a rectification circuit may be installed at the output side of the power generating unit.

Some components of the electronic device 1 may be shared by the modules. For example, the MPUs of the modules may be configured to access the storage unit 108. A storage region of the storage unit 108 may be divided into a plurality of regions, and the plurality of regions may be allocated as storage regions dedicated for the respective modules. The storage regions may be configured to be used in a time division manner by the respective modules. Alternatively, a control scheme in which the respective modules sequentially use the allocated storage regions may be applied to the electronic device 1.

### <Example of system using electronic device>

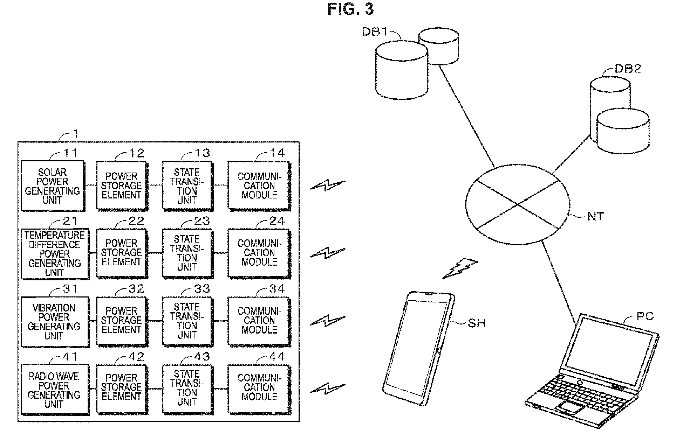

FIG. 3 illustrates an example of a system using the electronic device. As described above, the electronic device 1 performs communication with an external device using the communication modules of the respective modules. An external device is, for example, a database device DB1, a database device DB2, or a personal computer PC connected to the electronic device 1 via a network NT. The external device may be a device used in a company or a device used by an individual. The external device may be a device connected to the electronic device 1 via a cable.

The electronic device 1 may perform communication with the external device via a relay device. A smartphone SH that is owned by the user and located at a relatively close position to the electronic device may be used as the relay device. The relay device may be a tablet computer or a laptop personal computer in addition to a smartphone or may be one electronic device 1 when there is a plurality of electronic devices 1. The relay device is also the external device. A specific example (an application example) of the system using the electronic device will be described later.

### <Example of flow of process of electronic device>

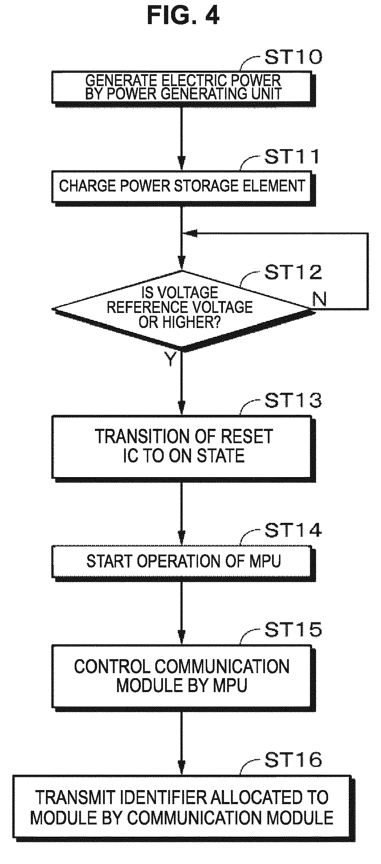

An example of the flow of a process of the modules in the electronic device 1 will be described with reference to a flowchart of FIG. 4. The following description will proceed with an example of the flow of a process of the module 10a. The flows of processes of the other modules are substantially the same as that of the module 10a, but there may be a difference according to the configuration of the module. For example, the processes of the respective modules are independently performed.

In step ST10, the power generating unit 100 (in this example, the solar power generating unit 11) generates electric power. For example, when the user wearing the electronic device 1 goes outside under fine weather, the power generating unit 100 is irradiated with sunlight, and thus the power generating unit 100 generates electric power. Of course, when the outdoor weather is rainy or cloudy, the power generating unit 100 does not generate electric power or generates slight electric power. Then, the process proceeds to step ST11.

In step ST11, the electric power generated by the power generating unit 100 is supplied to the capacitor 105 serving as one of the power storage elements through the regulator IC 101 or the like. Then, the capacitor 105 is charged, and the voltage of the capacitor 105 is increased. Then, the process proceeds to step ST12.

In step ST12, it is determined whether the voltage of the capacitor 105 is the reference voltage or higher. When the voltage of the capacitor 105 is smaller than the reference voltage, the process returns to step ST12. When the voltage of the capacitor 105 is the reference voltage or higher, the process proceeds to step ST13.

In step ST13, as the voltage of the capacitor 105 becomes the reference voltage or higher, the reset IC 106 transitions from the OFF state to the ON state. Further, as the voltage of the capacitor 105 becomes the reference voltage or higher, the state of the reset IC 106 transitions, and the determination process of step ST12 is not performed by a certain functional block. As the reset IC 106 transitions to the ON state, the output voltage of the capacitor 105 is supplied to the MPU 107. Then, the process proceeds to step ST14.

In step ST14, the MPU 107 operates using the electric power supplied from the capacitor 105 as a power source. For example, the MPU 107 reads a program stored in the ROM 108a, and performs a process according to a code described in the program. Then, the process proceeds to step ST15.

In step ST15, the MPU 107 supplies the electric power to the communication module 109 and controls the communication module 109. In other words, the MPU 107 instructs the communication module 109 to start communication and to transmit, for example, the identifier of the module 10a to an external device. Then, the process proceeds to step ST16.

In step ST16, the communication module 109 performs communication according to the control by the MPU 107. The communication module 109 transmits, for example, the identifier allocated to the module 10a to the external device according to a predetermined communication scheme. The external device that has received the identifier of the module 10a can recognize that, for example, the solar power generating unit 11 including the module 10a has generated an energy amount for operating all or a part of the system.

Although not illustrated, as the MPU 107 operates, the voltage of the capacitor 105 is decreased, and thus the reset IC 106 transitions from the ON state to the OFF state. As the power supply to the MPU 107 is interrupted, the operations of the MPU 107 and the communication module 109 are suspended. For example, when the power generation of the solar power generating unit 11 is continued, the above process is repeated.

The above process is performed as the power generating unit generates a predetermined amount or more of electric power. In other words, for example, when a minimum electric power for operating the MPU and the communication module is generated or charged, system (module)-specific information may be generated and output. The user of the electronic device need not perform a special operation. In the electronic device, the power generation state of the power generating unit is used as sensing information. In other words, the power generating unit functions as a sensor and a power source. Thus, it is unnecessary to install a unique configuration (for example, a sensor such as an acceleration sensor, a gyro sensor, a temperature sensor, or a geomagnetic sensor) for obtaining the sensing information.

Since the sensor and an AD converting unit for processing the sensing information need not be installed, it is unnecessary to mount a large battery cell for supplying electric power, and thus the size of the electronic device can be reduced. Since the size of the electronic device can be reduced, for example, the user does not feel discomfort even when the user wears the electronic device. Further, since the size of the electronic device can be reduced, the electronic device can be easily incorporated into another device. In the electronic device according to the embodiment, it is possible to prevent its operation from being suspended by a dead battery that may occur in small electronic devices.

### <1-2. Modified example of first embodiment>

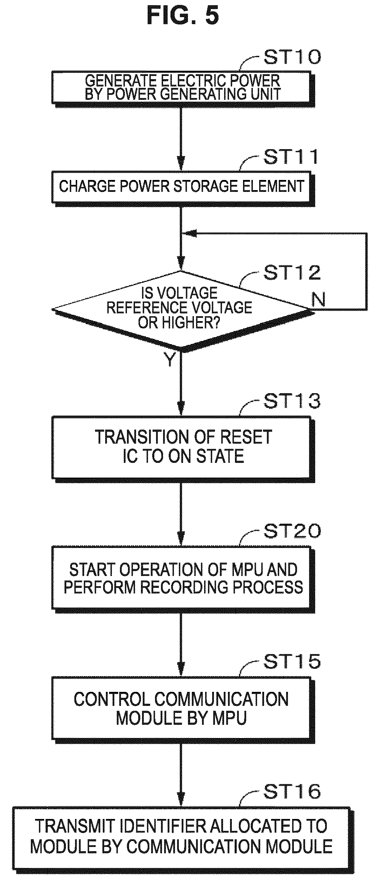

The process of the electronic device 1 according to the first embodiment can be modified, for example, as illustrated in a flowchart of FIG. 5. The following description will proceed with different points from the above process.

The process of step ST10 to step ST13 has been described above and thus will be described briefly. As the voltage equal to or higher than the reference voltage is charged in the capacitor 105, the reset IC 106 transitions from the OFF state to the ON state. Then, the process proceeds from step ST13 to step ST20.

As the reset IC 106 transitions to the ON state, the MPU 107 starts its operation. The MPU 107 stores information indicating that the reset IC 106 has transitioned to the ON state in the RAM 108b. The MPU 107 stores, for example, the identifier of the module 10a in the RAM 108b. Then, the MPU 107 reads the identifier of the module 10a stored in the RAM 108b, and instructs the communication module 109 to transmit the identifier to an external device. The process of step ST15 and step ST16 has been described already, and thus a duplicate description thereof will be omitted.

Further, information different from the identifier of the module 10a may be stored in the storage unit 108. For example, a counter may be installed in the storage unit 108, and the number of transitions of the reset IC 106 from the OFF state to the ON state may be stored. Then, the number of transitions of the module 10a to the ON state may be transmitted to the external device together with the identifier of the module 10a.

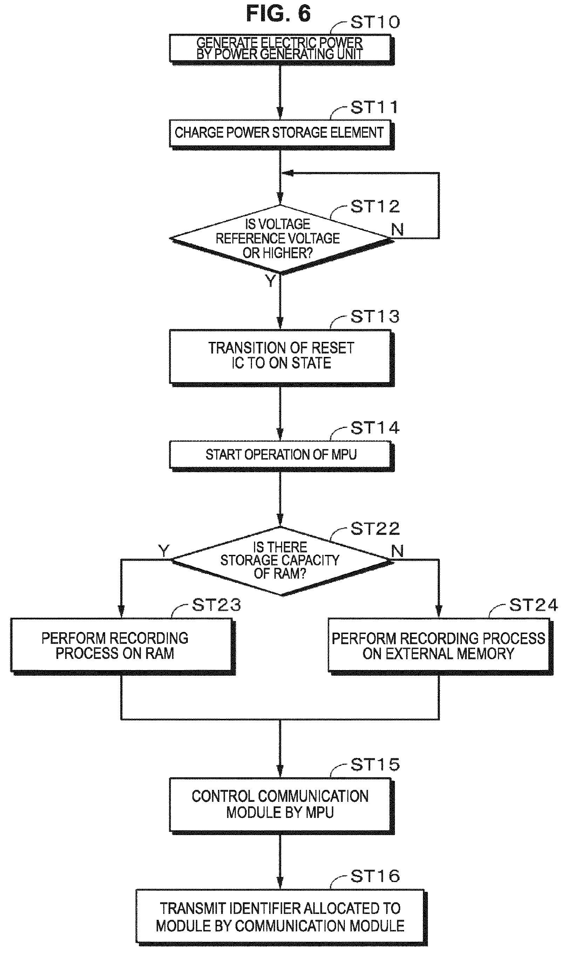

Further, when the MPU 107 stores the identifier of the module 10a or the like in the RAM 108b, process of step ST22, step ST23, and step ST24 in a flowchart of FIG. 6 may be performed.

In step ST22, the MPU 107 determines whether there is a storage capacity of the RAM 108b when a recording process is performed. When there is a storage capacity of the RAM 108b in step ST22, the process proceeds to step ST23. In step ST23, the MPU 107 performs the recording process on the RAM 108b. When there is not a storage capacity of the RAM 108b in step ST22, the process proceeds to step ST24.

In step ST24, since there is no storage capacity of the RAM 108b, the recording process is performed on an external memory. As will be described later in detail, an extension module having various functions may be added to the electronic device. When the extension module has a storage function, the identifier of the module 10a or the like may be stored in the extension module. The MPU 107 may determine whether the extension module has the storage function and perform the recording process on the extension module when the extension module has the storage function. Further, when there is no storage capacity of the RAM 108b, an overwriting process may be performed.

### <2. Second embodiment>

Next, a second embodiment will be described. In the description of the second embodiment, a duplicate description of the same components as in the first embodiment will appropriately be omitted. An electronic device according to the second embodiment may be configured to output time information indicating a time at which the state of the state transition unit transitions.

### <Example of configuration of electronic device>

The electronic device according to the second embodiment (referred to appropriately as an "electronic device 2") includes one or more of modules, similarly to the electronic device 1 according to the first embodiment. The electronic device 2 includes, for example, a module 10b, a module 20b, a module 30b, and a module 40b. Each of the modules includes, for example, a power generating unit, a power storage element, a state transition unit, and a communication module.

An example of a specific configuration of the module in the electronic device 2 will be described in connection with an example of the module 10b. For example, configurations of the other modules in the electronic device 2 are the same as the configuration of the module 10b, but there may be a difference in a configuration between the modules.

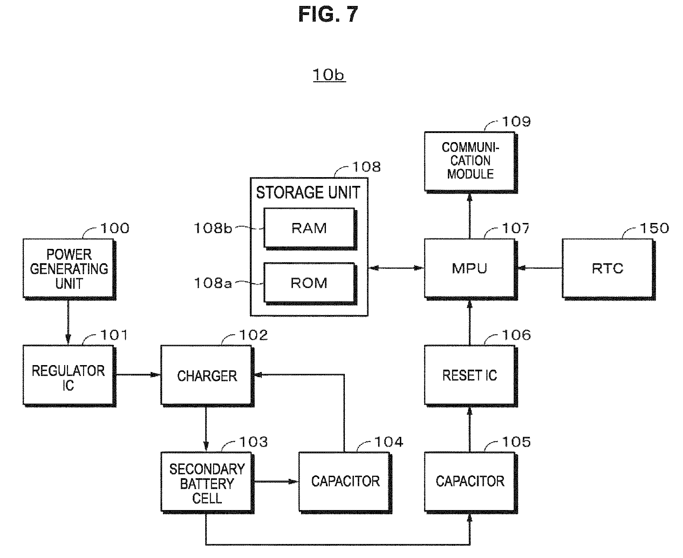

FIG. 7 illustrates an example of a specific configuration of the module 10b. Similarly to the module 10a in the electronic device 1, the module 10b has a configuration including, for example, a power generating unit 100, a regulator IC 101, a charger 102, a secondary battery cell 103, a capacitor 104, a capacitor 105, a reset IC 106, an MPU 107, a storage unit 108, and a communication module 109. The storage unit 108 has a configuration including, for example, a ROM 108a and a RAM 108b.

The module 10b includes a real time clock (RTC) 150. The RTC 150 is connected to the MPU 107, and supplies time information to the MPU 107. For example, a year, a month, and a date may be appropriately set to the time information. The MPU 107 may be configured to include the RTC therein.

Although not illustrated, a power source device for driving the RTC 150 is installed in the electronic device 2. For example, a secondary battery cell such as a lithium-ion battery cell or a primary battery cell may be used as the power source device. Since power consumption of the RTC 150 is small, a coin type small battery cell may be used. The electric power charged in the secondary battery cell 103 may be supplied to the RTC 150. As the reset IC 106 transitions from the OFF state to the ON state, the MPU 107 supplies the time information supplied from the RTC 150 to the communication module 109 together with the identifier of the module 10b. The communication module 109 outputs the identifier of the module 10b and the time information to an external device through communication.

### <Example of flow of process of electronic device>

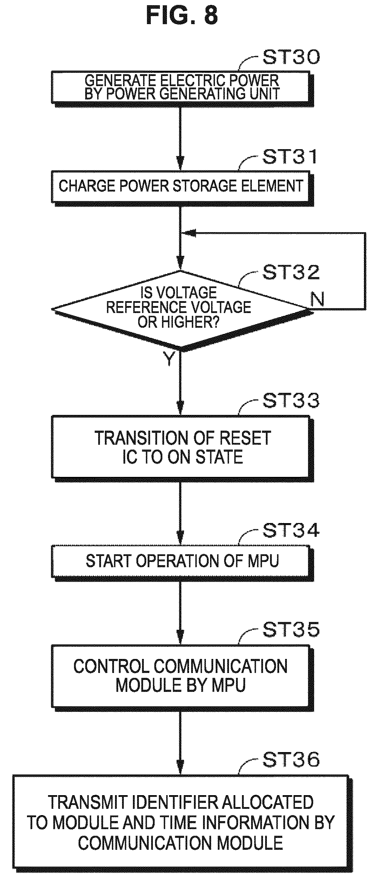

An example of the flow of a process of the modules in the electronic device 2 will be described with reference to a flowchart of FIG. 8. The following description will proceed with an example of the flow of a process of the module 10b. The flows of processes of the other modules are substantially the same as that of the module 10b, but there may be a difference according to the configuration of the module. For example, the processes of the respective modules are independently performed.

In step ST30, the power generating unit 100 (in this example, the solar power generating unit 11) generates electric power. For example, when the user wearing the electronic device 2 goes outside under fine weather, the power generating unit 100 is irradiated with sunlight, and thus the power generating unit 100 generates electric power. Of course, when the outdoor weather is rainy or cloudy, the power generating unit 100 does not generate electric power or generates slight electric power. Then, the process proceeds to step ST31.

In step ST31, the electric power generated by the power generating unit 100 is supplied to the capacitor 105 serving as one of the power storage elements through the regulator IC 101 or the like. Then, the capacitor 105 is charged, and the voltage of the capacitor 105 is increased. Then, the process proceeds to step ST32.

In step ST32, it is determined whether the voltage of the capacitor 105 is the reference voltage or higher. When the voltage of the capacitor 105 is smaller than the reference voltage, the process returns to step ST32. When the voltage of the capacitor 105 is the reference voltage or higher, the process proceeds to step ST33.

In step ST33, as the voltage of the capacitor 105 becomes the reference voltage or higher, the reset IC 106 transitions from the OFF state to the ON state. Further, as the voltage of the capacitor 105 becomes the reference voltage or higher, the state of the reset IC 106 transitions, and the determination process of step ST32 is not performed by a certain functional block. As the reset IC 106 transitions to the ON state, the output voltage of the capacitor 105 is supplied to the MPU 107. Then, the process proceeds to step ST34.

In step ST34, the MPU 107 operates using the electric power supplied from the capacitor 105 as a power source. The MPU 107, for example, reads a program stored in the ROM 108a, and performs a process according to a code described in the program. Then, the process proceeds to step ST35.

In step ST35, the MPU 107 supplies the electric power to the communication module 109 and controls the communication module 109. The MPU 107 supplies, for example, the identifier of the module 10b stored in the ROM 108a and the time information supplied from the RTC 150 to the communication module 109, and gives an instruction to output the information. Then, the process proceeds to step ST36.

In step ST36, the communication module 109 performs communication according to the control by the MPU 107. The communication module 109 transmits the identifier allocated to the module 10b and the time information to the external device according to a predetermined communication scheme. For example, the external device can recognize the power generation of the solar power generating unit 11 arranged in the module 10b and a power generation timing of the solar power generating unit 11. Further, when the external device receives the identifier of the module 10b and the time information again, the external device can recognize time intervals at which the state transition unit transitions to the ON state, that is, power generation intervals of the solar power generating unit 11 without using the time information at an external device side or without generating the time information.

The MPU 107 may store the time information indicating a time at which the reset IC 106 transitioned to the ON state in the RAM 108b. Thus, for example, even when communication is performed in a state in which information is accumulated instead of performing communication each time, a notification of the time information related to the state transition can be given to the external device side.

Although not illustrated, as the MPU 107 operates, the voltage of the capacitor 105 is decreased, and thus the reset IC 106 transitions from the ON state to the OFF state. As the power supply to the MPU 107 is interrupted, the operations of the MPU 107 and the communication module 109 are suspended. For example, when the power generation of the solar power generating unit 11 is continued, the above process is repeated.

### <3. Third embodiment>

Next, a third embodiment will be described. In the description of the third embodiment, a duplicate description of the same components as in the first embodiment will appropriately be omitted.

### <Example of configuration of electronic device>

The electronic device according to the third embodiment (referred to appropriately as an "electronic device 3") includes one or more of modules, similarly to the electronic device 1 according to the first embodiment. The electronic device 3 includes, for example, a module 10c, a module 20c, a module 30c, and a module 40c. Each of the modules includes, for example, a power generating unit, a power storage element, a state transition unit, and a communication module.

An example of a specific configuration of the module in the electronic device 3 will be described in connection with an example of the module 10c. For example, configurations of the other modules in the electronic device 3 are the same as the configuration of the module 10c, but there may be a difference in a configuration between the modules.

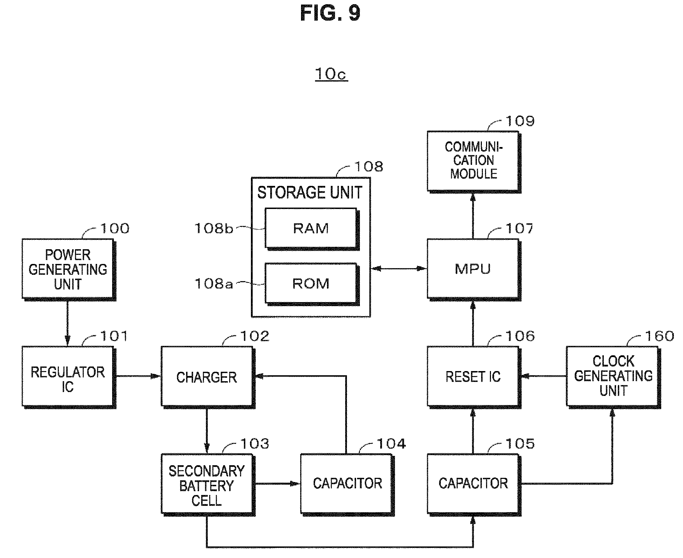

FIG. 9 illustrates an example of a specific configuration of the module 10c. Similarly to the module 10a in the electronic device 1, the module 10c has a configuration including, for example, a power generating unit 100, a regulator IC 101, a charger 102, a secondary battery cell 103, a capacitor 104, a capacitor 105, a reset IC 106, an MPU 107, a storage unit 108, and a communication module 109. The storage unit 108 has a configuration including, for example, a ROM 108a and a RAM 108b.

The module 10c further includes a clock generating unit 160. The clock generating unit 160 generates a clock, for example, with a predetermined cycle. When the clock generated by the clock generating unit 160 becomes a predetermined set value, the clock generating unit 160 performs control such that the output voltage of the capacitor 105 is supplied to the reset IC 106. For example, a switch is installed between the capacitor 105 and the reset IC 106, and when the clock becomes the predetermined set value, the clock generating unit 160 switches the switch from the OFF state to the ON state. The clock generating unit may be installed in a device different from the electronic device 3, and control may be performed by the clock generating unit outside the electronic device 3.

The electric power is supplied from the capacitor 105 to the clock generating unit 160. The clock generating unit 160 operates using the electric power supplied from the capacitor 105. A power source device for operating the clock generating unit 160 may be installed. For example, a secondary battery cell such as a lithium-ion battery cell or a primary battery cell may be used as the power source device. Since the power consumption of the clock generating unit 160 is small, a coin type small battery cell may be used. The electric power charged in the secondary battery cell 103 may be supplied to the clock generating unit 160.

### <Example of flow of process of electronic device>

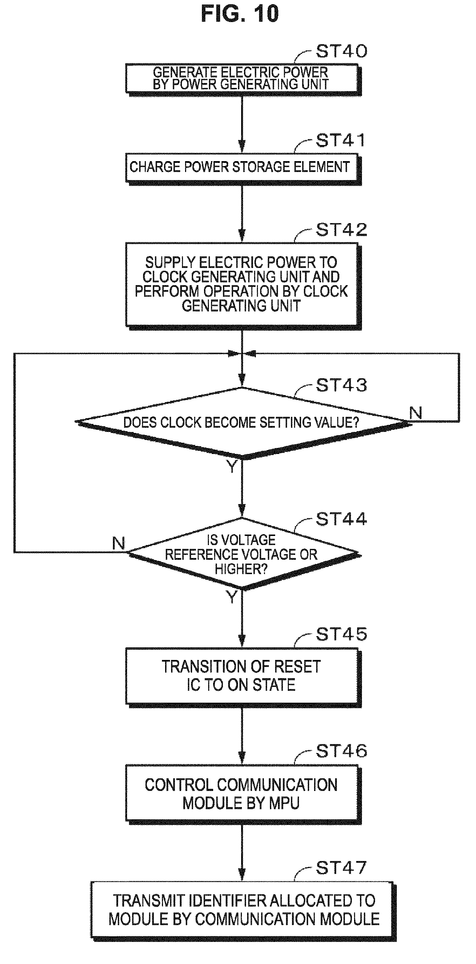

An example of the flow of a process of the modules in the electronic device 3 will be described with reference to a flowchart of FIG. 10. The following description will proceed with an example of the flow of a process of the module 10c. The flows of processes of the other modules are substantially the same as that of the module 10c, but there may be a difference according to the configuration of the module. For example, the processes of the respective modules are independently performed.

In step ST40, the power generating unit 100 (in this example, the solar power generating unit 11) generates electric power. For example, when the user wearing the electronic device 3 goes outside under fine weather, the power generating unit 100 is irradiated with sunlight, and thus the power generating unit 100 generates electric power. Of course, when the outdoor weather is rainy or cloudy, the power generating unit 100 does not generate electric power or generates slight electric power. Then, the process proceeds to step ST41.

In step ST41, the electric power generated by the power generating unit 100 is supplied to the capacitor 105 serving as one of the power storage elements through the regulator IC 101 or the like. Then, the capacitor 105 is charged, and the voltage of the capacitor 105 is increased. Then, the process proceeds to step ST42.

In step ST42, the electric power of the capacitor 105 is supplied to the clock generating unit 160. The clock generating unit 160 operates using the supplied electric power. Then, the process proceeds to step ST43.

In step ST43, the clock generating unit 160 determines whether the clock generated by the clock generating unit 160 has become a set value. When the clock has not become the set value, the process returns to step ST43. When the clock has become the set value, the clock generating unit 160 performs control such that the voltage of the capacitor 105 is supplied to the reset IC 106. Then, the process proceeds to step ST44.

In step ST44, it is determined whether the voltage of the capacitor 105 is the reference voltage or higher. When the voltage of the capacitor 105 is neither the reference voltage nor higher, the process returns to step ST43. Then, it is determined again whether the clock generated by the clock generating unit 160 becomes the set value. When the voltage of the capacitor 105 is the reference voltage or higher, the process proceeds to step ST46.

In step ST46, the MPU 107 operates using the electric power supplied from the capacitor 105 as a power source. The MPU 107 supplies the electric power to the communication module 109, and controls the communication module 109. The MPU 107 supplies, for example, the identifier of the module 10b stored in the ROM 108a to the communication module 109 and gives an instruction to output the information. Then, the process proceeds to step ST47.

In step ST47, the communication module 109 performs communication according to the control by the MPU 107. The communication module 109 transmits the identifier allocated to the module 10b to an external device according to a predetermined communication scheme.

Similarly to the modified example of the first embodiment, the MPU 107 may perform the recording process on the RAM 108b. Similarly to the second embodiment, the time information indicating a time at which the reset IC 106 transitioned to the ON state may be output from the RAM 108b to the external device.

Although not illustrated, as the MPU 107 operates, the voltage of the capacitor 105 is decreased, and thus the reset IC 106 transitions from the ON state to the OFF state. As the power supply to the MPU 107 is interrupted, the operations of the MPU 107 and the communication module 109 are suspended. For example, when the power generation of the solar power generating unit 11 is continued, the above process is repeated.

### <4. Content common to embodiments>

The configuration and operation of the electronic device of the present disclosure have been described using the electronic device 1, the electronic device 2, and the electronic device 3 as examples. Next, content that can be applied to the electronic devices in common will be described using the electronic device 1 as an example.

Since the electronic device 1 can be reduced in size, the electronic device 1 can be incorporated into another device or an accessory as an accessory, easily worn on an organism such as a person, an animal, or an outdoor tree, and carried. Examples of the accessory include a bracelet, a pendant, a ring, an earring, a hair band, and armor. The electronic device 1 may be attached to glasses, a hat, shoes, a bag, or the like usually used by the user. Of course, the electronic device 1 is not limited to one that is worn on an organism and carried. The electronic device 1 may be placed on a desk or may be placed on a thing (for example, a vehicle) other than an organism.

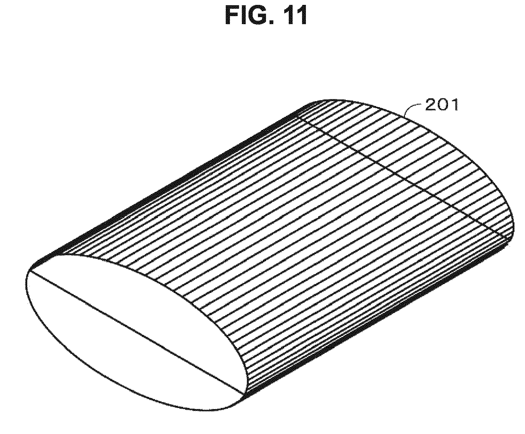

FIG. 11 illustrates an example of a housing in which the module (for example, the module 10a) in the electronic device 1 is accommodated. A housing 201 has, for example, a cross section of an elliptical shape. The size of the housing 201 can be appropriately set. As an example, the size of the housing 201 is set to a size of about a fingertip. The housing 201 is made of a light transmissive member such as glass or plastic so that the power generating unit accommodated in the housing 201 is irradiated with sunlight or indoor light.

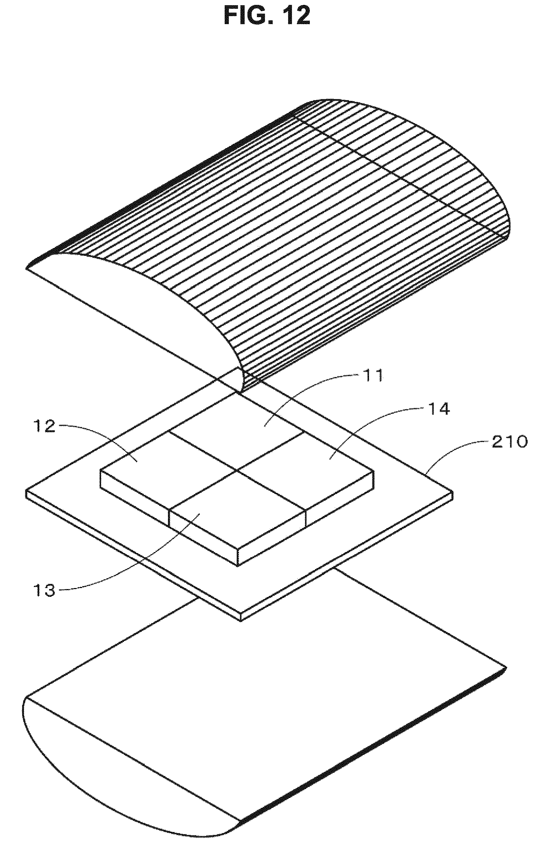

FIG. 12 an example of a simplified internal configuration of the housing 201. The respective components of the module 10a are accommodated in the housing 201. For example, a substrate 210 is accommodated in the housing 201, and the respective components of the module 10a are mounted on either or both of the surfaces of the substrate 210. The periphery of the module 10a may be molded by resin or the like. For example, the solar power generating unit 11, the power storage element 12, the state transition unit 13, and the communication module 14 including the MPU 107 are mounted on the substrate 210. In FIG. 12, for the sake of convenience of description, the housing 201 can be divided in two, but the housing 201 may be integrally molded not to be divided.

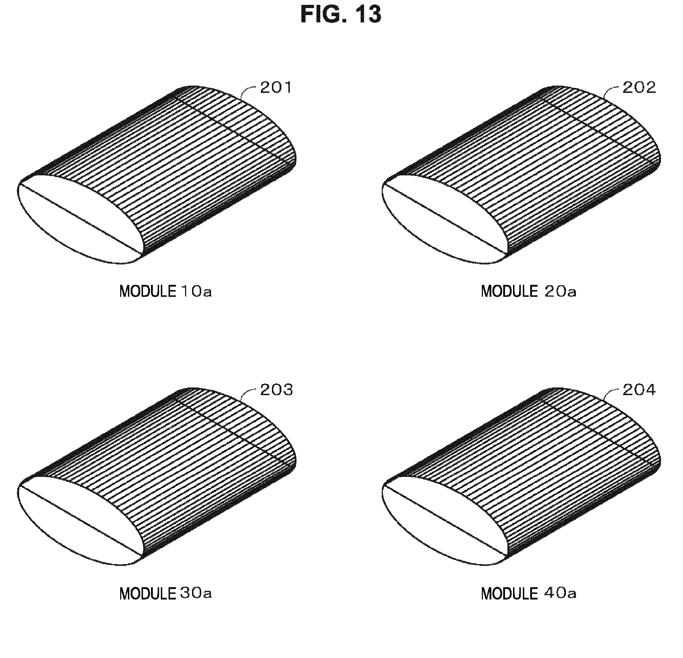

As illustrated in FIG. 13, the respective components of the module 20a are accommodated in a housing 202 having the same shape of the housing 201. The respective components of the module 30a are accommodated in a housing 203 having the same shape of the housing 201. The respective components of the module 40a are accommodated in a housing 204 having the same shape of the housing 201. In other words, in this example, the electronic device 1 includes the housings 201, 202, 203, and 204 in which the respective components of the modules are accommodated.

For example, by connecting the housings 201, 202, 203, and 204 in a circle using a lace or a chain and putting them around the neck of the user, the electronic device 1 can be used like a necklace. Further, by connecting the housings 201, 202, 203, and 204 using a circular band, the electronic device 1 can be used like a wristband. By further reducing the housings 201, 202, 203, and 204 in size and attaching the downsized housings to a ring-like metallic member, the electronic device 1 can be used like a ring. Of course, these use forms are examples and are not limiting.

Further, an arrangement position of the power generating unit in the housing may be set according to characteristics of the power generating unit. For example, when the power generating unit is the solar power generating unit, it is desirable to arrange the solar power generating unit at a surface side of the inside of the housing so that lights falls on the solar power generating unit. For example, when the power generating unit is the vibration power generating unit, it is desirable to arrange the vibration power generating unit near the center of the inside of the housing in order to reduce influence of noise. For example, when the power generating unit is configured with a piezoelectric element, it is desirable to arrange the piezoelectric element at the surface side of the inside of the housing.

### <Extension module>

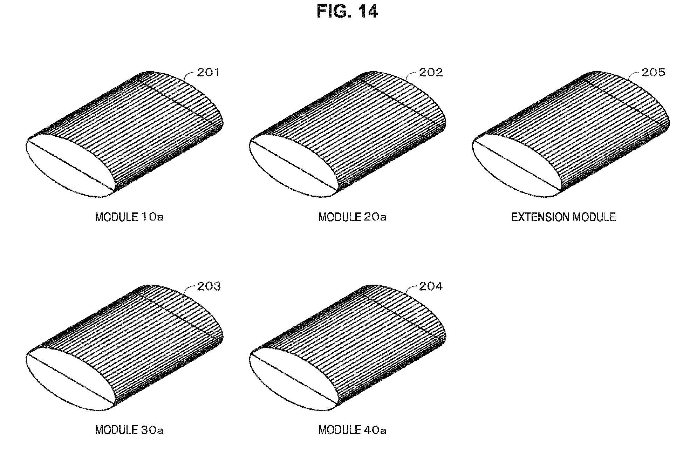

The function of the electronic device 1 can be extended, for example, using an extension module. As illustrated in FIG. 14, an extension module includes a housing 205 having the same shape as the housing 201, for example. A configuration for implementing the extension module is accommodated in the housing 205.

For example, by arranging a memory and a driver for performing a recording and reproducing process on the memory in the housing 205, it is possible to cause the extension module to function as a storage device. For example, by arranging an electronic paper or the like in the housing 205, it is possible to cause the extension module to function as a display. For example, by installing a switch or a button on the surface of the housing 205 and electrically connecting the housing 205 with the respective modules, it is possible to cause the extension module to function as a device that supplies some triggers to the respective modules.

For example, by arranging a clock generating device in the housing 205 and electrically connecting the housing 205 with the respective modules, it is possible to cause the extension module to function as a device that provides a clock to the respective modules. For example, by installing an MPU in the housing 205 and electrically connecting the housing 205 with the respective modules, it is possible to cause the extension module to function as a control device that controls the respective modules.

For example, by arranging a battery cell in the housing 205, it is possible to cause the extension module to function as a battery cell that supplies electric power to the respective modules. For example, by arranging a configuration implementing a clocking function and a display function in the housing 205, it is possible to cause the extension module to function as a clocking device. For example, by arranging a speaker in the housing 205, it is possible to cause the extension module to function as a speaker device. The extension module may be configured as a universal serial bus (USB) memory. For example, by forming a terminal of a predetermined shape in the housing 205, it is possible to cause the extension module to function as a terminal for connecting the electronic device 1 with another device.

For example, the inside of the housing 205 may be hollow or may be filled with resin or the like. It is possible to cause the extension module to function as an adjustment member for adjusting the size of the electronic device 1 to be suitable for the size of a neck or a hand, for example, when a plurality of modules are connected, and the electronic device 1 is used as an accessory. As described above, the electronic device 1 may be configured to include the extension module in addition to the modules. Further, a plurality of extension modules may be used in the electronic device 1.

By arranging a communication module and a power source for driving the communication module in the housing 205, it is possible to cause the extension module to function as a main communication module. The term "main" in the main communication module is an expression for convenience of description to distinguish it from the communication module in the module 10a or the like, and it does not have a special meaning.

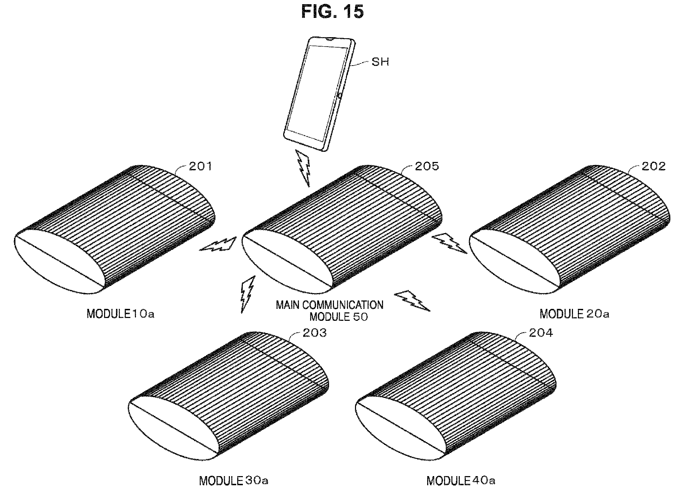

As illustrated in FIG. 15, for example, the respective modules (the module 10a, the module 20a, the module 30a and the module 40a) and the main communication module (referred to appropriately as a "main communication module 50") perform wired or wireless communication with one another. For example, NFC of several tens of centimeters is performed. Each of the modules transmits the allocated identifier or the like to the main communication module 50 as the state transition unit transitions to the ON state. The main communication module 50 transmits information of the identifier received from the modules to an external device such as the smartphone SH.

As described above, the communication with the external device may be performed through the main communication module. Here, an example of a communication system constructed by the electronic device and the external device will be described. In the above description, in order to facilitate a description, an illustration of the power storage element, the state transition unit, and the like will appropriately be omitted.

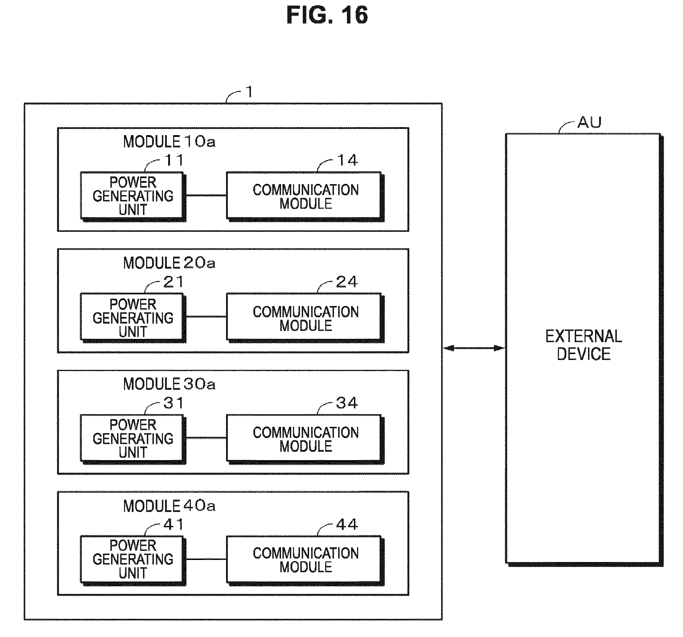

FIG. 16 is an example of a communication system constructed by the electronic device 1 and an external device AU. The example illustrated in FIG. 16 is an example corresponding to the above embodiment, and the main communication module is not used. In other words, the example illustrated in FIG. 16 is an example in which the communication module of each module independently performs communication with the external device AU.

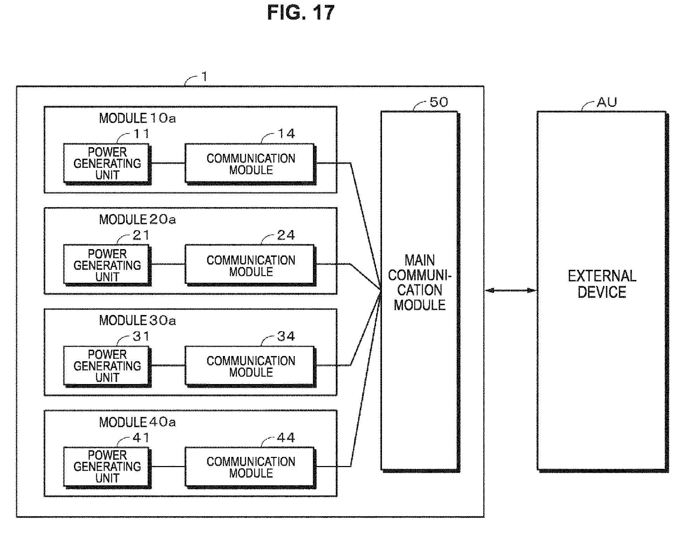

FIG. 17 illustrates another example of the communication system constructed by the electronic device 1 and the external device AU. The example illustrated in FIG. 17 is an example corresponding to the communication system described with reference to FIG. 15. Each module transmits the identifier of the module or the like to the main communication module 50 through NFC. The main communication module 50 transmits information received from the modules to the external device AU. Since it is desirable that the communication module of each module perform, for example, NFC, the size of the communication module or the antenna can be reduced. Further, since energy consumption for a series of communication operations is suppressed, a communication frequency can be increased. This corresponds to an improvement in sensitivity in sensor and temporal resolution, for example. Since the main communication module 50 needs a certain size but does not need a power generating unit or the like, it is possible to prevent the main communication module 50 from being increased in size.

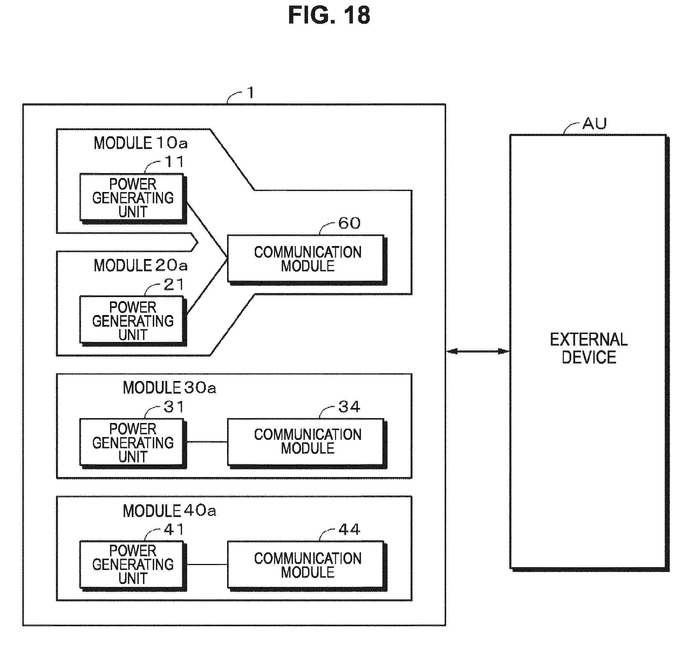

Further, the power generating units in the electronic device 1 may be grouped, and a communication module may be installed for each group. For example, the solar power generating unit 11 of the module 10a and the temperature difference power generating unit 21 of the module 20a are grouped as illustrated in FIG. 18. A communication module (a communication module 60) corresponding to the group is installed. In other words, the module 10a and the module 20a share the communication module 60. The present embodiment is not limited to the communication module, and one state transition unit corresponding to the group may be installed.

In the example illustrated in FIG. 18, for example, it is necessary to check whether the communication module 60 is being used by performing communication between the MPU of the module 10a and the MPU of the module 20a. For example, the MPU of the module 10a inquires to the MPU of the module 20a whether the communication module 60 is being used. Here, when the state transition unit of the module 20a is in the OFF state, and the MPU is not in an activated state, there is no response to the inquiry. Thus, the MPU of the module 10a controls the communication module 60 such that the identifier allocated to the module 10a or the like can be transmitted to the external device AU.

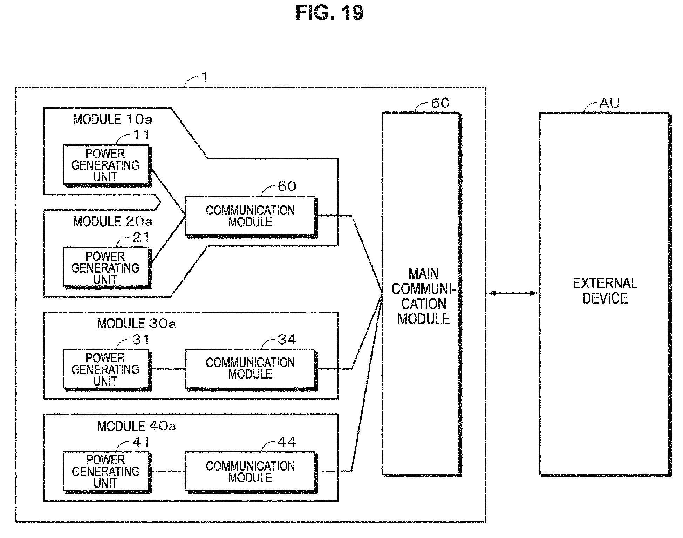

Further, as illustrated in FIG. 19, a communication module (for example, the communication module 60 in FIG. 18) shared by a plurality of modules may perform communication with the main communication module 50. For example, NFC may be performed between the communication module 60 and the main communication module 50.

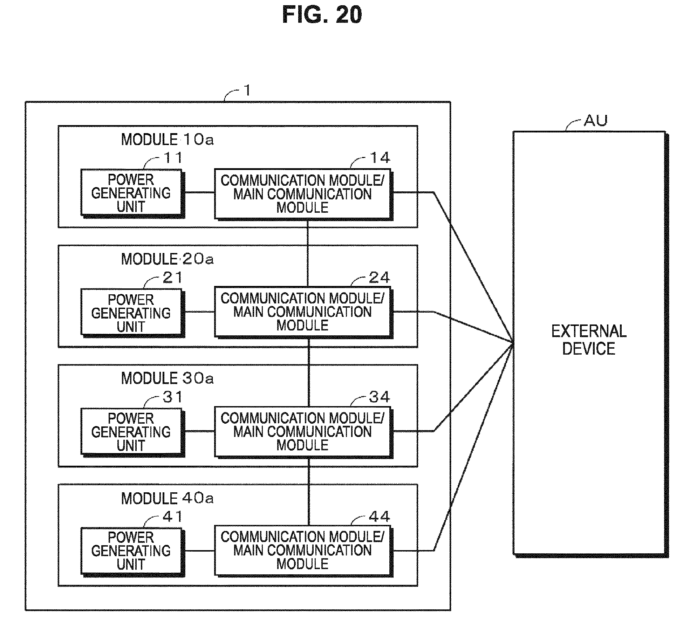

Further, as illustrated in FIG. 20, the communication module of each module may be configured to function as a main communication system according to circumstances. For example, the communication module of the module having the power generating unit having the largest power generation amount is allocated as the main communication module. For example, the power generation amount is specified by any one of a voltage, an electric current, electric power and electric energy or a combination thereof. Communication is performed between the communication module decided as the main communication module and another communication module. The main communication module transmits information received from another communication module to the external device.

In the configuration of the communication system illustrated in FIG. 20, it is necessary to monitor the power generation amount of each power generating unit. To this end, for example, it is desirable to monitor the power generation amount of the power generating unit and install an extension module (a control device) that controls whether any one communication module functions as the main communication module according to the monitoring result. However, it is unnecessary to install the main communication module as the extension module. The example of the communication system is not limited to the exemplified example and can be appropriately changed.

### <5. Application examples>

Next, application examples of the electronic device will be described. Of course, content of the present disclosure is not limited to application examples which will be described below.

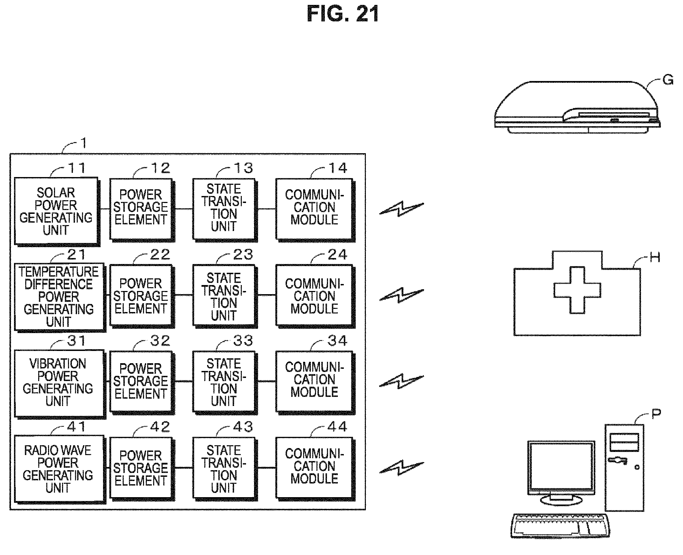

As illustrated in FIG. 21, the electronic device 1 (the electronic device 2 or the electronic device 3) outputs predetermined information to a second electronic device through communication or the like. For example, the predetermined information is the identifier allocated to each module or the time information. The second electronic device includes a receiving unit that receives the predetermined information transmitted from the electronic device 1 as an example of an acquiring unit. The second electronic device generates various information based on the predetermined information transmitted from the electronic device 1.

Examples of the second electronic device include a game machine G, a health management device H, and an analysis device. For example, the analysis device is a position information identification device P. The electronic device 1 may perform communication with the second electronic device via a relay device (a third electronic device) such as a smartphone. For example, the second electronic device already has information about a configuration of a power generating unit arranged in a module corresponding to the identifier. For this reason, for example, when the identifier of the module 10a is transmitted from the electronic device 1, the second electronic device can recognize that the solar power generating unit 11 of the module 10a has generated a predetermined amount or more of electric power.

### <Application example of game system>

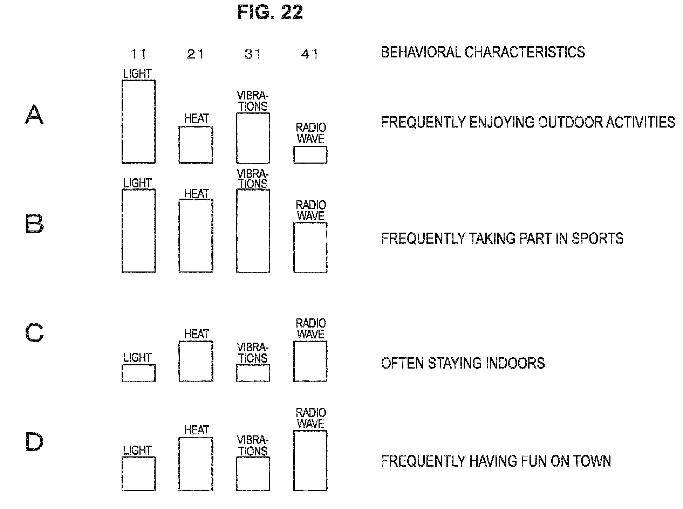

First, an application example of a game system in which the second electronic device functions as the game machine G will be described with reference to FIGS. 22A to 22D. The game machine G includes a control device such as a central processing unit (CPU) for implementing a function described below, a storage device that stores the number of receptions of the identifier of the module during a certain period of time, and the like. In this example, the game device may be a device having a game function such as a smartphone serving as an example of the relay device. In FIG. 22A to FIG. 22D, a bar graph schematically indicates a pattern of the number of receptions of the identifier of the module.

In an example illustrated in FIG. 22A, the number of receptions of the identifier of the module 10a including the solar power generating unit 11 is largest, and the number of receptions of the identifier of the module 30a including the vibration power generating unit 31 is next largest. The number of receptions of the identifier of the module 20a including the temperature difference power generating unit 21 is a predetermined number of times, and the number of receptions of the identifier of the module 40a including the radio wave power generating unit 41 is smallest.

In an example illustrated in FIG. 22B, the number of receptions of the identifier of the module 10a including the solar power generating unit 11 and the number of receptions of the identifier of the module 30a including the vibration power generating unit 31 are large. The number of receptions of the identifier of the module 20a including the temperature difference power generating unit 21 is large as well. The number of receptions of the identifier of the module 40a including the radio wave power generating unit 41 is also the predetermined number of times or more.

In an example illustrated in FIG. 22C, the number of receptions of the identifier of the module 10a including the solar power generating unit 11 and the number of receptions of the identifier of the module 30a including the vibration power generating unit 31 are small. The number of receptions of the identifier of the module 20a including the temperature difference power generating unit 21 and the number of receptions of the identifier of the module 40a including the radio wave power generating unit 41 are the predetermined number of times or more.

In an example illustrated in FIG. 22D, the number of receptions of the identifier of the module 10a including the solar power generating unit 11 and the number of receptions of the identifier of the module 30a including the vibration power generating unit 31 are the predetermined number of times or more. On the other hand, the number of receptions of the identifier of the module 20a including the temperature difference power generating unit 21 and the number of receptions of the identifier of the module 40a including the radio wave power generating unit 41 are larger than the number of receptions of the identifier of the module 10a.

The game machine G analyzes behavioral characteristics of the user of the electronic device 1 using this information. For example, in the case of the pattern of FIG. 22A, since the number of receptions of the identifier of the module 10a is large, the solar power generating unit 11 frequently generates electric power. In other words, the user of the electronic device 1 is inferred to have an outdoor activity. On the other hand, since the number of receptions of the identifier of the module 40a is small, the radio wave power generating unit 41 generates little electric power. In other words, the user of the electronic device 1 is inferred to have an activity in a region (for example, a mountain) which a radio wave hardly reaches.

Meanwhile, since the number of receptions of the identifier of the module 20a and the number of receptions of the identifier of the module 30a are at a normal level, the temperature difference power generating unit 21 and the vibration power generating unit 31 generate a normal level of electric power. In other words, the user is inferred to have an activity such as walking. Thus, in the case of the pattern of FIG. 22A, a behavioral characteristic of "frequently enjoying outdoor activities" is determined.

In the case of the pattern of FIG. 22B, since the number of receptions of the identifier of the module 10a is large, the solar power generating unit 11 frequently generates electric power. In other words, the user of the electronic device 1 is inferred to have an outdoor activity. Further, since the number of receptions of the identifier of the module 20a and the number of receptions of the identifier of the module 30a are also large, the temperature difference power generating unit 21 and the vibration power generating unit 31 frequently generate electric power. In other words, the user of the electronic device 1 is inferred to have a vigorous activity. Thus, in the case of the pattern of FIG. 22B, a behavioral characteristic of "frequently taking part in sports" is determined.

In the case of the pattern of FIG. 22C, since the number of receptions of the identifier of the module 10a and the number of receptions of the identifier of the module 30a are small, the solar power generating unit 11 and the vibration power generating unit 31 generate little electric power. In other words, the user of the electronic device 1 is inferred to stay indoors and not to have a vigorous activity. Thus, in the case of the pattern of FIG. 22C, a behavioral characteristic of "often staying indoors" is determined.

In the case of the pattern of FIG. 22D, since the number of receptions of the identifier of the module 20a is slightly large, the temperature difference power generating unit 21 generates electric power. In other words, the user of the electronic device 1 is inferred to have a slightly vigorous activity. Further, since the number of receptions of the identifier of the module 40a is large, the radio wave power generating unit 41 frequently generates electric power. In other words, the user of the electronic device 1 is inferred to stay in a place (for example, an urban area) which a radio wave reaches well. Thus, in the case of the pattern of FIG. 22D, a behavioral characteristic of "frequently having fun on the town" is determined.

The game machine G generates information related to a game, such as characters used in a game, based on the determined behavioral characteristic. For example, when the behavioral characteristic of "frequently enjoying outdoor activities" is determined, for example, a "character living in a mountain" bunting an outdoor activity to mind is generated by reflecting the behavioral characteristic. For example, when the behavioral characteristic of "frequently taking part in sports" is determined, for example, a "character of a tough warrior" is generated by reflecting the behavioral characteristic.

For example, when the behavioral characteristic of "often staying indoors" is determined, for example, a "character of an intelligent magician" brining an image of enjoy studying or acquisition of knowledge to mind is generated by reflecting the behavioral characteristic. For example, when the behavioral characteristic of "frequently having fun on the town" is determined, for example, a "character of a mischievous elf" brining a character liking to play to mind is generated by reflecting the behavioral characteristic.

The game machine G is set so that the generated character can be used in the game. The game machine G may transmit data of the generated character to a smartphone of the user or the like. As described above, it is possible to generate the information related to the game using the information transmitted from the electronic device 1. Further, the information may be used for an avatar of an application or the like other than a game.

### <Application example of health management system>

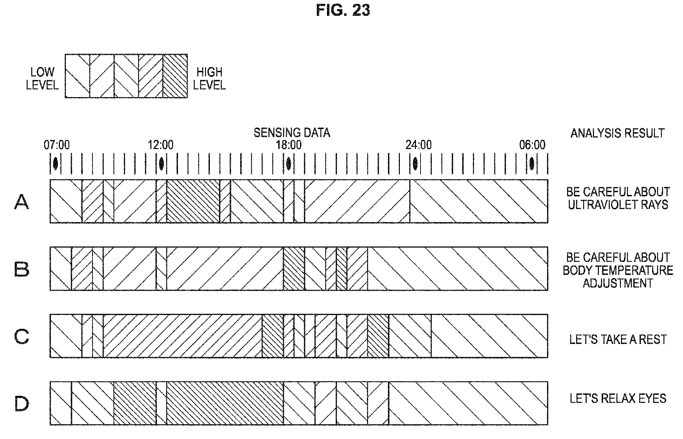

Next, an example in which the electronic device 1 is applied to a health management system will be described with reference to FIGS. 23A to 23D. The electronic device 1 transmits the information such as the identifier of the module to the health management device H. The health management device H includes, for example, a control device such as a CPU for implementing a function described below. The health management device H generates information related to health, for example, advice contributing to health management using the predetermined information transmitted from the electronic device 1.

FIG. 23A illustrates an example of a change in the number of receptions of the identifier of the module 10a during one day. The number of receptions is set to, for example, five levels, and the respective levels are indicated by different hatchings. A time zone indicated by a high level hatching indicates that the number of receptions of the identifier of the module 10a is large. As illustrated in FIG. 23A, the number of receptions changes to a relatively high level from about 8:00 am to the evening. The health management device H analyzes that the solar power generating unit 11 frequently generates electric power since the user of the electronic device 1 frequently goes out based on the change in the number of receptions. The health management device H generates advice for health management according to the analysis result. For example, the health management device H generates the advice of "be careful about ultraviolet rays."

FIG. 23B illustrates an example of a change in the number of receptions of the identifier of the module 20a during one day. A time zone indicated by a high level hatching indicates that the number of receptions of the identifier of the module 20a is large. As illustrated in FIG. 23B, in the morning and the evening, the number of receptions changes to a relatively high level. The health management device H analyzes that the temperature difference power generating unit 21 generates electric power since the user of the electronic device 1 comes and goes between the outdoors and indoors in which there is a big temperature difference to commute or study during the summer or the winter based on the change in the number of receptions. The health management device H generates advice for health management according to the analysis result. For example, the health management device H generates the advice of "be careful about body temperature adjustment."

FIG. 23C illustrates an example of a change in the number of receptions of the identifier of the module 30a during one day. A time zone indicated by a high level hatching indicates that the number of receptions of the identifier of the module 30a is large. As illustrated in FIG. 23C, excluding a period from the night to the morning, the number of receptions changes at a relatively high level. The health management device H analyzes that the vibration power generating unit 31 frequently generates electric power since the user of the electronic device 1 takes part in vigorous activities based on the change in the number of receptions. The health management device H generates advice for health management according to the analysis result. For example, the health management device H generates the advice of "let's take a rest."

FIG. 23D illustrates an example of a change in the number of receptions of the identifier of the module 40a during one day. A time zone indicated by a high level hatching indicates that the number of receptions of the identifier of the module 40a is large. As illustrated in FIG. 23D, the number of receptions changes at a relatively high level during daylight. The health management device H analyzes that the radio wave power generating unit 41 frequently generates electric power using a radio wave generated from a personal computer since the user of the electronic device 1 works on the personal computer during daylight based on the change in the number of receptions. The health management device H generates advice for health management according to the analysis result. For example, the health management device H generates the advice of "let's relax eyes."

For example, the health management device H transmits the generated advice to an address of an e-mail which is registered in advance. The user can get the advice for health management. The advice generated by the health management device H may be transmitted to the user by mail or the like.

### <Application example of authentication system>

Next, an example in which the electronic device 1 is applied to the authentication system will be described. For example, the authentication system includes the electronic device 1, an authentication information determination system Y, and a key Z that is at least one of a physical key and a logical key. The authentication information determination system Y includes a control device such as a CPU for implementing a function described below. The electronic device 1 generates at least one of identification information identifying an individual on which the electronic device 1 is mounted or installed and identification information identifying a group serving as a set of individuals on which the electronic device 1 is mounted or installed.

The authentication information determination system Y includes a database. The database stores a feature of information identifying an individual transmitted from the electronic device 1 for each individual, each group, or each condition. For example, as described above in the example of the health management system, according to an environment in which a target (referred to appropriately as a "mounting/installation target") on which the electronic device 1 is mounted or installed is placed, the electronic device 1 indirectly generates information indicating a feature of the mounting/installation target. In other words, even though it is sufficient or insufficient in defining information specifying the mounting/installation target or a specific condition to be satisfied, information generated from various kinds of energy which the electronic device 1 obtains from the surrounding environment of the mounting/installation target as effective information is used as authentication information A.

Further, by intentionally applying input energy to the electronic device 1 through the mounting/installation target using a certain pattern, it is possible to cause the electronic device 1 to generate information specific to the mounting/installation target. In other words, for example, it is possible to intentionally generate feature information by repeating an action of blocking sunlight from reaching the electronic device 1 that generates information when receiving sunlight during a certain period of time at a certain timing with a hand. This feature quantity can be used as information that can be detected by only the mounting/installation target that has generated it or information defining a certain condition to be satisfied. Thus, even though it is sufficient or insufficient in defining information specifying the mounting/installation target or a specific condition, information which the electronic device 1 generates as effective information is used as authentication information B.

The authentication information A, the authentication information B, or a combination of the authentication information A and B may be stored in the database as authentication information. Further, as the information specifying the mounting/installation target or the information defining a specific condition to be satisfied, a combination of information that is not generated by the electronic device 1 and information that is generated by the electronic device 1 may be used. In other words, there is a feature quantity specific to the mounting/installation target, for example, a physical feature quantity (a fingerprint, a voiceprint, an iris, a vein, DNA, a contour of a face or a body, or the like) in the case of a human being, ID information such as a character string, information related to an interest or a preference, and the like, and a combination of authentication information C obtained therefrom and either or both of the authentication information A and B obtained from the electronic device 1 can be used as authentication information D.

For example, one or more of the authentication information A, B, C, and D is registered in the database with which the authentication information determination system Y is equipped, and by comparing the registered authentication information with the information generated by the electronic device 1 or additional information generated from the information generated by the electronic device 1, the physical or logical key z opens or closes.

A form of the key Z is not consequential, for example, the key Z may be a door that is physically locked, a contact point that is locked by an electric signal or the like, a variable used for access control from a certain program to another program, or the like. As a more specific example, when an attribute value, an occupation, or the like is allocated to the mounting/installation target through information from the electronic device 1 mounted or installed on the mounting/installation target as in the application example of the game system, a zone on a game program which only a certain specific attribute or occupation is allowed to enter may be set, and entrance may be limited in a real space other than a game program.

Further, for example, a bracelet-like accessory form made by partially using the electronic device 1 may be used to change according to a specific condition, for example, such that a vibration/motion power generation device installed in the electronic device 1 generates electric power in a specific power generation pattern, for example, by rotating the accessory, and a form in which power generation based on sunlight is performed once or more per second or a color is changed. It may be used for controlling the mounting/installation target, for example, an event intended only for the mounting/installation target satisfying a specific condition may be performed.

As in the application example of the health management system, a health condition of the mounting/installation target may be detected according to information from the electronic device 1 mounted or installed on the mounting/installation target. Then, grouping may be performed according to the health condition of the mounting/installation target, and, for example, an access zone of a hospital ward may be classified according to the group, or an accessible medicine case may be controlled according to the group.

### <Application example of position information identification system>

Next, an example in which the electronic device 1 is applied to the position information identification system will be described. The position information identification system includes, for example, the electronic device 1 and a position information identification device P. The position information identification device P includes a control device such as a CPU for implementing a function described below.

The position information identification device P includes a database. The database stores a feature of information transmitted from the electronic device 1 for each region. For example, a region A is assumed to be a region in which the weather is mostly fine, a temperature difference is small, and reception of a radio wave is not fine. In this region, since the solar power generating unit 11 frequently generates electric power, the number of receptions of the identifier of the module 10a tends to be large. On the other hand, since the temperature difference power generating unit 21 and the radio wave power generating unit 41 generate little electric power, the number of receptions of the identifiers of the module 20a and the module 40a tends to be small.

Further, for example, a region B is assumed to be a region in which the weather is mostly rainy, a temperature difference is large, and a road is chaotic. In this region, since the solar power generating unit 11 generates little electric power, the number of receptions of the identifier of the module 10a tends to be small. On the other hand, since the temperature difference power generating unit 21 and the vibration power generating unit 31 are likely to generate a relatively large amount of electric power, the number of receptions of the identifiers of the module 20a and the module 30a tends to be large.

The position information identification device P searches for a region having a tendency which is identical or similar to the tendency of the number of receptions of the identifier of the module transmitted from the electronic device 1 from information accumulated in the database. Then, a region in which the electronic device 1 is located is determined based on the search result.

### <Other application examples>

Information usable in a plurality of websites may be generated according to predetermined information transmitted from the electronic device 1. Further, information related to a power generation capacity of the power generating unit may be generated. For example, demonstration data indicating an amount of electric power that can be generated by the solar power generating unit, the radio wave power generating unit, or a newly developed power generating unit may be generated. For example, the demonstration data may be provided to a company that has developed the power generating unit or an electric utility company.

Further, secondary information obtained by estimating a power generation capacity (an electric energy, average electric power, an average voltage, or the like) of the power generating unit of the module may be generated using the number of receptions of the identifier of the module or a reception time interval. Furthermore, tertiary information such as an amount of sunlight when the power generating unit of the module is the solar power generating unit, a temperature or a metabolic calorie when the power generating unit of the module is the temperature difference power generating unit, acceleration or momentum when the power generating unit of the module is the vibration power generating unit, or radio wave strength when the power generating unit of the module is the radio wave power generating unit may be generated using the secondary information.

Instead of the number of receptions of the identifier of the module, the identifier of the module and the time information transmitted from the electronic device 1 may be used. The game machine G or the like can determine that the power generating unit of the module frequently generates electric power when the interval of the time information is short.

### <6. Modified examples>

The embodiments of the present disclosure have specifically been described, but the present disclosure is not limited to the above embodiments, and various kinds of modifications can be made based on the technical spirit of the present disclosure.

In the above embodiments, the state transition unit of the electronic device is configured to cause the state to transition based on the electric energy, but the embodiment is not limited thereto. The state transition unit causes the state to transition based on different energy (a first energy) from electric energy. In this case, the first energy is converted into electric energy serving as an example of a second energy, the state of the state transition unit transitions, and then electric energy is supplied to an output unit (for example, the communication module). The output unit outputs the predetermined information to an external device using the supplied electric energy. A specific example of the predetermined information has been described above in the first embodiment, and thus a duplicate description will be omitted.

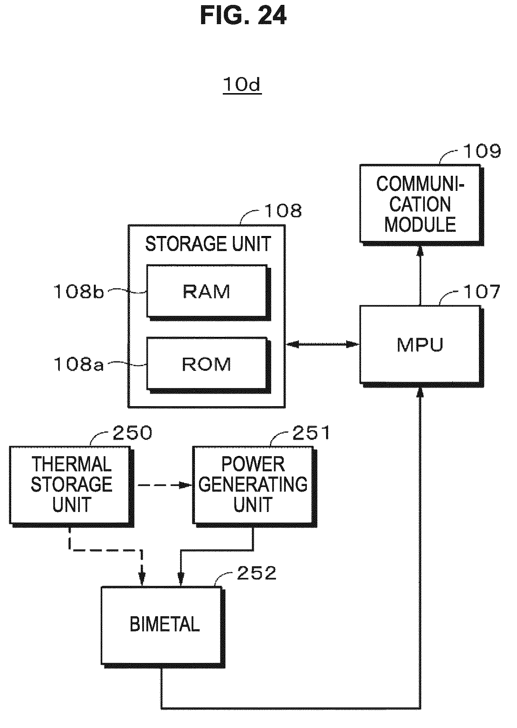

FIG. 24 illustrates an example of a configuration of a module (referred to appropriately as a "module 10d") of the electronic device according to a modified example. In FIG. 24, the flow of the first energy is indicated by a dotted line, and the flow of the second energy or a signal or a command based on the second energy is indicated by a solid line to be distinguished. The same components as those in the module 10a are denoted by the same reference numerals, and a duplicate description will appropriately be omitted.

The module 10d includes a thermal storage unit 250 that accumulates thermal energy serving as an example of the first energy, a power generating unit 251 serving as an example of a converting unit, and a bimetal 252 serving as an example of a state transition unit. The module 10d further includes an MPU 107, a storage unit 108, and a communication module 109.

The thermal storage unit 250 accumulates the thermal energy, and supplies the thermal energy to the power generating unit 251 and the bimetal 252 at an appropriate timing. The power generating unit 251 is a device that converts the thermal energy supplied from the thermal storage unit 250 into electric energy, and a known device may be applied. The bimetal 252 is a switch that is configured with a bimetal obtained by joining metals having different thermal expansions and a contact point, and supplies or interrupts electricity as the bimetal expands due to a temperature change to operate the contact point. For example, the bimetal 252 transitions between two states of ON and OFF, and when the bimetal 252 is in the ON state, the bimetal 252 is electrically connected to the MPU 107.

An example of an operation of the module 10d will briefly be described. Through a thermal storage operation of the thermal storage unit 250, the thermal storage unit 250 accumulates the thermal energy. The thermal energy is supplied from the thermal storage unit 250 to the power generating unit 251 and the bimetal 252. The power generating unit 251 generates electric power by converting the supplied thermal energy into electric energy. The temperature of the bimetal 252 increases according to the supplied thermal energy, and a state in which the temperature thereof becomes a predetermined temperature or more is the ON state. As the bimetal 252 enters the ON state, the bimetal 252 is electrically connected to the MPU 107.

The bimetal 252 may be used for ON/OFF switching of a thermal contact point instead of ON/OFF switching of an electric contact point. In other words, when the bimetal 252 enters the ON state with the increase in the temperature of the thermal storage unit 250, the bimetal 252 is thermally connected to the power generating unit 251, and the thermal energy is conducted from the thermal storage unit 250 through the bimetal 252. The power generating unit 251 may be configured to generate electric power according to the inflow thermal energy at this time.

As the bimetal 252 is electrically connected to the MPU 107 or as the power generating unit 251 is directly connected to the MPU 107, the electric power generated by the power generating unit 251 is supplied to the MPU 107. The MPU 107 supplies the electric power to the storage unit 108 and the communication module 109, and performs control such that the communication module 109 performs a predetermined operation. The communication module 109 operates according to the control by the MPU 107, and the communication module 109 transmits, for example, the identifier of the module 10d to an external device. A power storage element such as a capacitor may be installed between the MPU 107 and either the bimetal 252 or the power generating unit 251, and the electric power generated by the power generating unit 251 may be stored in the power storage element.

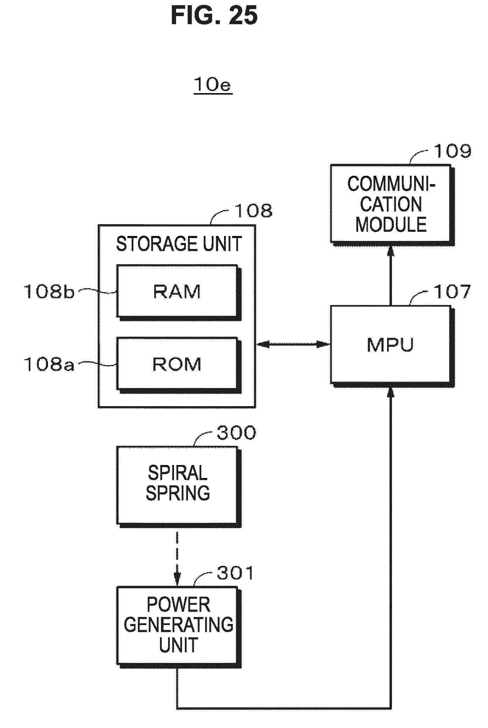

Another example will be described. FIG. 25 illustrates an example of a configuration of a module (referred to appropriately as a "module 10e") of the electronic device according to a modified example. In FIG. 25, the flow of the first energy is indicated by a dotted line, and the flow of the second energy or a signal or a command based on the second energy is indicated by a solid line to be distinguished. The same components as those in the module 10a are denoted by the same reference numerals, and a duplicate description will appropriately be omitted.

The module 10e includes a spiral spring 300 and a power generating unit 301 serving as an example of a converting unit. The module 10e further includes an MPU 107, a storage unit 108, and a communication module 109. The spiral spring 300 functions as a power storage unit that accumulates kinetic energy applied from the outside and functions as a state transition unit that transitions between a state in which the accumulated kinetic energy is accumulated and a state in which the accumulated kinetic energy is released. The state in which the kinetic energy is accumulated refers to, for example, a state in which the spiral spring 300 is sufficiently wound up, and the state in which the kinetic energy is released refers to, for example, a state in which the wound spiral spring 300 performs a rewinding operation. Further, for example, vibration of the user wearing the electronic device 1 is supplied to the spiral spring 300 as the kinetic energy.

The power generating unit 301 is, for example, a mechanism that includes a magnet and a coil and performs electromagnetic induction power generation. As one of the magnet and the coil of the power generating unit 301 rotates according to the kinetic energy supplied from the spiral spring 300, electric power is generated. The electric power generated by the power generating unit 301 is supplied to the MPU 107.

An example of an operation of the module 10e will briefly be described. Through the vibration of the user or the like, the spiral spring 300 is wound up, and the kinetic energy is accumulated in the spiral spring 300. Then, after the spiral spring 300 is sufficiently wound, the spiral spring 300 enters the release state, and thus the kinetic energy is supplied to the power generating unit 301. Due to the kinetic energy supplied by the spiral spring 300, for example, the magnet of the power generating unit 301 rotates, and the power generating unit 301 generates electric power. The electric power generated by the power generating unit 301 is supplied to the MPU 107.

The MPU 107 supplies the electric power to the storage unit 108 and the communication module 109, and performs control such that the communication module 109 performs a predetermined operation. The communication module 109 operates according to the control by the MPU 107, and the communication module 109 transmits, for example, the identifier of the module 10e to an external device. Further, a power storage element such as a capacitor may be installed between the power generating unit 301 and the MPU 107, and the electric power generated by the power generating unit 301 may be stored in the power storage element.

In addition to the above examples, a light storage material that accumulates optical energy may be used as an element that stores energy in a non-electric form. In this case, an energy converting device that converts optical energy into electric energy is used.

In the above embodiments, when the state transition unit transitions to the ON state, the identifier of the module or the like is transmitted to the external device, but only the recording process may be performed by the MPU when the state transition unit transitions to the ON state. For example, the number of times in which the state transition unit transitions to the ON state is stored in the storage unit of the electronic device. For example, the information stored in the storage unit of the electronic device is output to a personal computer when the electronic device is connected to the personal computer.

The present disclosure may be implemented by a method, a program, a system, or the like without being limited to a device. For example, the program may be provided to the user via a network or a portable memory such as an optical disk or a semiconductor memory.

In this case, a form of the program is not consequential as long as a function of the program is implemented, for example, the program may be an object code, a program executed by an interpreter, script data supplied to an OS, or the like. As a method of supplying a program using wired or wireless communication, a method of storing a computer program for implementing content of the present disclosure or a data file (a program data file) serving as a computer program for implementing content of the present disclosure on a client computer such as a compressed file with an automatic installation function in a server on a computer network and downloading the program data file to a connected client computer may be used. In this case, the program data file may be divided into a plurality of segment files, and the segment files may be arranged in different servers.

The configurations and the processes of the embodiments and the modified examples may be appropriately combined within the scope in which no technical contradiction occurs. The order of the processes in the flow of the process may be appropriately combined within the scope in which no technical contradiction occurs.

The present disclosure can be applied to a cloud system in which the above-described processes are distributed and performed by a plurality of devices. The present disclosure can be implemented as a device in which at least some of the above-described processes are performed in a system in which the above-described processes according to the embodiments and the modified example are performed.

Additionally, the present technology may also be configured as below.
(1) An electronic device, including:
   a power generating unit configured to generate electric power according to a surrounding environment;
   a state transition unit configured to cause a state to transition according to the electric power supplied from the power generating unit; and
   an output unit configured to output predetermined information according to the transition of the state of the state transition unit.
(2) The electronic device according to (1), including:
   a recording unit configured to record the predetermined information,
   wherein the output unit reads the recorded predetermined information and then outputs the predetermined information.
(3) The electronic device according to (1) or (2),
   wherein, according to the transition of the state of the state transition unit, the electric power generated by the power generating unit is supplied to the output unit.
(4) The electronic device according to (3), including:
   a control unit connected to the state transition unit and the output unit,
   wherein, according to the transition of the state of the state transition unit, the electric power generated by the power generating unit is supplied to the control unit, and
   the output unit outputs the predetermined information according to control by the control unit.
(5) The electronic device according to any of (1) to (4),
   wherein the electric power generated by the power generating unit is supplied to the state transition unit via one or more power storage elements.
(6) The electronic device according to any of (1) to (5),
   wherein it is determined whether the state of the state transition unit has transitioned with a predetermined cycle.
(7) The electronic device according to any of (1) to (6),
   wherein the predetermined information is an identifier allocated to the power generating unit.
(8) The electronic device according to (7),
   wherein the identifier is allocated to the power generating unit when communication with an external device is established.
(9) The electronic device according to any of (1) to (8),
   wherein the predetermined information includes the identifier and time information when the state of the state transition unit has transitioned.
(10) The electronic device according to any of (1) to (9),
   wherein the output unit outputs the predetermined information through wired or wireless communication.
(11) The electronic device according to any of (1) to (10), including:
   a main output unit,
   wherein communication is performed between the output unit and the main output unit, and
   the main output unit outputs the predetermined information transmitted from the output unit to an external device.
(12) The electronic device according to (11),
   wherein wired communication is performed between the output unit and the main output unit.
(13) The electronic device according to (11),
   wherein near field communication is performed between the output unit and the main output unit.
(14) The electronic device according to any of (1) to (13),
   wherein the electronic device is configured to be worn on a human body or an animal and be portable.
(15) The electronic device according to any of (1) to (14),
   wherein the power generating unit generates electric power by energy based on any one of light, heat, vibrations, a radio wave, and an enzyme or energy obtained by getting close to a predetermined device.
(16) The electronic device according to any of (1) to (15),
   wherein the state transition unit is a circuit or an element that transitions from an OFF state to an ON state when a power generation amount output from the power generating unit is a predetermined amount or more.
(17) The electronic device according to (16),
   wherein the power generation amount is specified by any one of a voltage, an electric current, electric power and electric energy or a combination thereof.
(18) The electronic device according to any of (1) to (17), including:
   a plurality of the power generating units.
(19) The electronic device according to (18),
   wherein the state transition unit and the output unit are installed for each of the plurality of power generating units.
(20) The electronic device according to any of (1) to (19),
   wherein the output unit that performs communication with an external device is decided according to a power generation amount of each of the plurality of power generating units.
(21) The electronic device according to (20),
   wherein the power generation amount is specified by any one of a voltage, an electric current, electric power and electric energy or a combination thereof.
(22) The electronic device according to (20) or (21),
   wherein communication is performed between the decided output unit and another output unit, and the decided output unit outputs the predetermined information transmitted from the another output unit to the external device.
(23) The electronic device according to any of (18) to (22),
   wherein the plurality of power generating units are grouped, and
   the state transition unit and the output unit are installed for each group of the power generating units.
(24) The electronic device according to any of (1) to (23), including:
   a housing configured to accommodate the power generating unit therein,
   wherein a position of the power generating unit in the housing is set according to a characteristic of the power generating unit.
(25) The electronic device according to any of (1) to (24),
   wherein the electronic device is configured to have at least one of a clock function, a display function, and a terminal function.
(26) An electronic device, including:
   a power storage unit configured to accumulate a first energy;
   a state transition unit configured to cause a state to transition according to the first energy;
   a converting unit configured to convert the first energy into a second energy; and
   an output unit configured to output predetermined information using the second energy, the second energy being supplied according to the transition of the state of the state transition unit.
(27) An information processing method in an electronic device, the information processing method including:
   generating, by a power generating unit, electric power according to a surrounding environment;
   causing, by a state transition unit, a state to transition according to the electric power supplied from the power generating unit; and
   outputting, by an output unit, predetermined information according to the transition of the state of the state transition unit.
(28) An information processing system, including:
   a first electronic device; and
   a second electronic device,
   wherein the first electronic device includes
   a power generating unit configured to generate electric power according to a surrounding environment,
   a state transition unit configured to cause a state to transition according to the electric power supplied from the power generating unit, and
   an output unit configured to output predetermined information to the second electronic device according to the transition of the state of the state transition unit, and
   the second electronic device includes
   an acquiring unit configured to acquire the predetermined information output from the first electronic device.
(29) The information processing system according to (28), including:
   a third electronic device configured to perform communication with the first electronic device and the second electronic device,
   wherein the predetermined information output from the first electronic device is supplied to the second electronic device through the third electronic device.
(30) The information processing system according to (28) or (29),
   wherein the second electronic device generates information based on at least one of a frequency at which the predetermined information is supplied and an interval at which the predetermined information is supplied.
(31) The information processing system according to any of (28) to (30),
   wherein the second electronic device generates any one of information usable in a plurality of websites, information related to a game, information related to health, information related to authentication, information related to a position at which the first electronic device stays, and information related to a power generation capacity of the power generating unit, based on at least one of a frequency at which the predetermined information is supplied and an interval at which the predetermined information is supplied.

### Reference Signs List

- 1: electronic device
- 10a, 10b, 10c, 10d, 10e: module
- 11: solar power generating unit
- 21: temperature difference power generating unit
- 31: vibration power generating unit
- 41: radio wave power generating unit
- 13, 23, 33, 43: state transition unit
- 14, 24, 34, 44: communication module
- 100, 201, 301: power generating unit
- 106: reset IC
- 107: MPU
- 108: storage unit
- 109: communication module
- 200: thermal storage unit
- 202: bimetal
- 300: spiral spring

## Claims

1. An electronic device, comprising:
a power generating unit configured to generate electric power according to a surrounding environment;
a state transition unit configured to cause a state to transition according to the electric power supplied from the power generating unit; and
an output unit configured to output predetermined information according to the transition of the state of the state transition unit.

2. The electronic device according to claim 1, comprising:
a recording unit configured to record the predetermined information,
wherein the output unit reads the recorded predetermined information and then outputs the predetermined information.

3. The electronic device according to claim 1,
wherein, according to the transition of the state of the state transition unit, the electric power generated by the power generating unit is supplied to the output unit.

4. The electronic device according to claim 3, comprising:
a control unit connected to the state transition unit and the output unit, wherein, according to the transition of the state of the state transition unit, the electric power generated by the power generating unit is supplied to the control unit, and
the output unit outputs the predetermined information according to control by the control unit.

5. The electronic device according to claim 1,
wherein the electric power generated by the power generating unit is supplied to the state transition unit via one or more power storage elements.

6. The electronic device according to claim 1,
wherein it is determined whether the state of the state transition unit has transitioned with a predetermined cycle.

7. The electronic device according to claim 1,
wherein the predetermined information is an identifier allocated to the power generating unit.

8. The electronic device according to claim 7,
wherein the identifier is allocated to the power generating unit when communication with an external device is established.

9. The electronic device according to claim 7,
wherein the predetermined information includes the identifier and time information when the state of the state transition unit has transitioned.

10. The electronic device according to claim 1,
wherein the output unit outputs the predetermined information through wired or wireless communication.

11. The electronic device according to claim 1, comprising:
a main output unit,
wherein communication is performed between the output unit and the main output unit, and
the main output unit outputs the predetermined information transmitted from the output unit to an external device.

12. The electronic device according to claim 11,
wherein wired communication is performed between the output unit and the main output unit.

13. The electronic device according to claim 11,
wherein near field communication is performed between the output unit and the main output unit.

14. The electronic device according to claim 1,
wherein the electronic device is configured to be worn on a human body or an animal and be portable.

15. The electronic device according to claim 1,
wherein the power generating unit generates electric power by energy based on any one of light, heat, vibrations, a radio wave, and an enzyme or energy obtained by getting close to a predetermined device.

16. The electronic device according to claim 1,
wherein the state transition unit is a circuit or an element that transitions from an OFF state to an ON state when a power generation amount output from the power generating unit is a predetermined amount or more.

17. The electronic device according to claim 16,
wherein the power generation amount is specified by any one of a voltage, an electric current, electric power and electric energy or a combination thereof.

18. The electronic device according to claim 1, comprising:
a plurality of the power generating units.

19. The electronic device according to claim 18,
wherein the state transition unit and the output unit are installed for each of the plurality of power generating units.

20. The electronic device according to claim 18,
wherein the output unit that performs communication with an external device is decided according to a power generation amount of each of the plurality of power generating units.

21. The electronic device according to claim 20,
wherein the power generation amount is specified by any one of a voltage, an electric current, electric power and electric energy or a combination thereof.

22. The electronic device according to claim 20,
wherein communication is performed between the decided output unit and another output unit, and the decided output unit outputs the predetermined information transmitted from the another output unit to the external device.

23. The electronic device according to claim 18,
wherein the plurality of power generating units are grouped, and
the state transition unit and the output unit are installed for each group of the power generating units.

24. The electronic device according to claim 1, comprising:
a housing configured to accommodate the power generating unit therein,
wherein a position of the power generating unit in the housing is set according to a characteristic of the power generating unit.

25. The electronic device according to claim 1,
wherein the electronic device is configured to have at least one of a clock function, a display function, and a terminal function.

26. An electronic device, comprising:
a power storage unit configured to accumulate a first energy;
a state transition unit configured to cause a state to transition according to the first energy;
a converting unit configured to convert the first energy into a second energy; and
an output unit configured to output predetermined information using the second energy, the second energy being supplied according to the transition of the state of the state transition unit.

27. An information processing method in an electronic device, the information processing method comprising:
generating, by a power generating unit, electric power according to a surrounding environment;
causing, by a state transition unit, a state to transition according to the electric power supplied from the power generating unit; and
outputting, by an output unit, predetermined information according to the transition of the state of the state transition unit.

28. An information processing system, comprising:
a first electronic device; and
a second electronic device,
wherein the first electronic device includes
a power generating unit configured to generate electric power according to a surrounding environment,
a state transition unit configured to cause a state to transition according to the electric power supplied from the power generating unit, and
an output unit configured to output predetermined information to the second electronic device according to the transition of the state of the state transition unit, and
the second electronic device includes
an acquiring unit configured to acquire the predetermined information output from the first electronic device.

29. The information processing system according to claim 28, comprising:
a third electronic device configured to perform communication with the first electronic device and the second electronic device,
wherein the predetermined information output from the first electronic device is supplied to the second electronic device through the third electronic device.

30. The information processing system according to claim 28,
wherein the second electronic device generates information based on at least one of a frequency at which the predetermined information is supplied and an interval at which the predetermined information is supplied.

31. The information processing system according to claim 28,
wherein the second electronic device generates any one of information usable in a plurality of websites, information related to a game, information related to health, information related to authentication, information related to a position at which the first electronic device stays, and information related to a power generation capacity of the power generating unit, based on at least one of a frequency at which the predetermined information is supplied and an interval at which the predetermined information is supplied.
